# EUROPÄISCHE PATENTANMELDUNG

(11) **EP 2 762 571 A1**
(43) Veröffentlichungstag der Anmeldung: **06.08.2014**
(21) Anmeldenummer: 13153211.1
(22) Anmeldetag: 30.01.2013
(51) Int. Cl.: C12P 13/08, C12P 13/12, C12N 9/02

(54) **Mikroorganismus und Verfahren zur fermentativen Herstellung von Aminosäuren**

(71) Anmelder: Evonik Industries AG, 45128 Essen (DE)
(72) Erfinder: Walter, Frederik, 33602 Bielefeld (DE); Dr. Persicke, Marcus, 33647 Bielefeld (DE); Dr. Kalinowski, Jörn, 33615 Bielefeld (DE); Dr. Hüser, Andrea, 33615 Bielefeld (DE); Dr. Claes, Wilfried, 33619 Bielefeld (DE); Reth, Alexander, 33615 Bielefeld (DE)

(57) **Zusammenfassung**

Die Erfindung betrifft einen Mikroorganismus, der eine organisch-chemische Verbindung produziert und/oder ausscheidet, wobei der Mikroorganismus eine erhöhte Expression im Vergleich zu dem jeweiligen Ausgangsstamm von einem Polypeptid LpdA mit der Aktivität einer Transhydrogenase aufweist; sowie ein Verfahren zur Herstellung einer organisch-chemischen Verbindung unter Verwendung des erfindungsgemäßen Mikroorganismus.

SEQUENCE LISTING

<110>  Evonik Industries AG

<120>  Mikroorganismus und Verfahren zur fermentativen Herstellung von
       Aminosäuren

<130>  2011E00472

<160>  9

<170>  PatentIn version 3.5

<210>  1
<211>  3410
<212>  DNA
<213>  Corynebacterium glutamicum


<220>
<221>  CDS
<222>  (1001)..(2410)
<223>  lpdA gene

<400>  1
acggtgatcg ctttggcact tgcctgtgca ctgaacacca tcgaactgcc catcggcatt      60

cgggtgattt tccagccggc agaagaagtc atgactggtg gcgcaacgga cgtcattgcc     120

cacggtggcc ttgatggtgt ggatgcgatt tacgccatcc acgttgaacc caaattgaag     180

gtcggtcgcg tcggtgtacg cgctggcgcg attacttctg cctcagatgt gatcgaaatc     240

agagtcaagg gtgaaggagg acatagcgca cgtccacacc tctccgctga tgttgtttac     300

gccttgagca aattggtcgt tgatcttccc ggtttgctgt ccaggcgcgt cgatccacgc     360

accggcaccg tgcttgtttt cggcaccatc aacgccggct atgcgcccaa cgcgatccca     420

gattccggca tcgtgtcagg caccttgcgt acagccgaca tctctacctg gcgtgacatg     480

cgtccgctta tctctgagct ggtggaacag gtgctcgcac ccaccggagt cacccatgaa     540

ctgatctaca atccgggtgt tccaccagtg cttaacgacg atgtcgccac cgctttgttg     600

gcaagcgcag cacgcgacat ggacacacaa tctgttgtcc aagcgccgca gtcatccggt     660

ggagaagact tctcgtggta ccttgaacac gtcccaggat caatggcccg gttgggttgc     720

tggccggggc acggacccaa gcaagacctc catcaaagtg acctggttgt ggatgagcga     780

gccatcggag ttggcgtcag gctctttggc tcccttgtgc agcagtacag tagccgatct     840

gaagctttct taaattccta atgggggtag tgtgtagggc tggctctaaa ttgctgacac     900

gtcacccagg ctaattcagc agtaatcatt tagacttgga accgcttacc agtggtttca     960

acaatgcatt cacccagctc acacgtgtgg aggtgcctta atg gca aag agg atc      1015
                                           Met Ala Lys Arg Ile
                                           1               5

gta att atc ggc ggt gga cct gca ggc tat gaa gcc gca ctc gca ggc      1063
Val Ile Ile Gly Gly Gly Pro Ala Gly Tyr Glu Ala Ala Leu Ala Gly
                10                  15                  20

gct aaa tac ggt gca gaa gtt acc gtt att gaa gat gtc gga gtt ggc 1111
Ala Lys Tyr Gly Ala Glu Val Thr Val Ile Glu Asp Val Gly Val Gly
            25                  30                  35

gga tcc gca gtc acc atg gac tgt gta cct tca aag tcc ttc atc gct 1159
Gly Ser Ala Val Thr Met Asp Cys Val Pro Ser Lys Ser Phe Ile Ala
        40                  45                  50

ggt acc ggt atc aaa acc gac ctc cga cgt gct gat gac atg gga ctt 1207
Gly Thr Gly Ile Lys Thr Asp Leu Arg Arg Ala Asp Asp Met Gly Leu
    55                  60                  65

aac cgt ggg ctt gga aaa gca cac cta gaa atc gat gca ctg aac atc 1255
Asn Arg Gly Leu Gly Lys Ala His Leu Glu Ile Asp Ala Leu Asn Ile
70                  75                  80                  85

cgt gtg aag gac ctt gcg aaa gca cag tcc gaa gat atc ttg ggc cag 1303
Arg Val Lys Asp Leu Ala Lys Ala Gln Ser Glu Asp Ile Leu Gly Gln
                90                  95                  100

ctg cag cgc tca gat gtc cgc atg att aac ggt gtg ggc cgc ttt gat 1351
Leu Gln Arg Ser Asp Val Arg Met Ile Asn Gly Val Gly Arg Phe Asp
            105                 110                 115

gat tac aac acc aag caa acc acc cac tac att aaa gtc acc cac agc 1399
Asp Tyr Asn Thr Lys Gln Thr Thr His Tyr Ile Lys Val Thr His Ser
        120                 125                 130

gat ggc tcc gaa gaa acc gtt gag tgc gat ctg gtg ctg gtt gca act 1447
Asp Gly Ser Glu Glu Thr Val Glu Cys Asp Leu Val Leu Val Ala Thr
    135                 140                 145

ggt gca acc ccc cgc att ctt aaa ggt gca gag cca gac ggc gag cgc 1495
Gly Ala Thr Pro Arg Ile Leu Lys Gly Ala Glu Pro Asp Gly Glu Arg
150                 155                 160                 165

atc ttg acc tgg cgt cag gtc tac gac att gaa gaa ctc ccc acc cac 1543
Ile Leu Thr Trp Arg Gln Val Tyr Asp Ile Glu Glu Leu Pro Thr His
                170                 175                 180

ctt atc gtg gtt ggt tcc ggt gtg acc ggt gcg gaa ttt gtc tct gcg 1591
Leu Ile Val Val Gly Ser Gly Val Thr Gly Ala Glu Phe Val Ser Ala
            185                 190                 195

ttt gct gaa ctc ggc gtc aaa gtc acc atg gtg gca tcc cgt gac cgc 1639
Phe Ala Glu Leu Gly Val Lys Val Thr Met Val Ala Ser Arg Asp Arg
        200                 205                 210

att ttg cct cac gat gac gca gat gcc gca gac gtg ctg gaa acc gtt 1687
Ile Leu Pro His Asp Asp Ala Asp Ala Ala Asp Val Leu Glu Thr Val
    215                 220                 225

ctg gct gag cgc gga gta tcc ctg gaa aag cat gcc cgc gtg gag tct 1735
Leu Ala Glu Arg Gly Val Ser Leu Glu Lys His Ala Arg Val Glu Ser
230                 235                 240                 245

gtc acc cgc acc gaa gac ggt ggc gtg tgt gtt cgc act gct gac gga 1783
Val Thr Arg Thr Glu Asp Gly Gly Val Cys Val Arg Thr Ala Asp Gly
                250                 255                 260

cga gaa atc tac ggt tct cac gcg ttg atg act gtt ggt tcc att cca 1831
Arg Glu Ile Tyr Gly Ser His Ala Leu Met Thr Val Gly Ser Ile Pro
            265                 270                 275

aac acg gca gat ctt ggc ctg gag aac atc ggt gtt gag ctg gca cca 1879

Asn Thr Ala Asp Leu Gly Leu Glu Asn Ile Gly Val Glu Leu Ala Pro
        280                 285                 290

tcc ggc cat atc aag gtt gac cgc gtc tcc cgc acc aac atc ccc ggt        1927
Ser Gly His Ile Lys Val Asp Arg Val Ser Arg Thr Asn Ile Pro Gly
    295                 300                 305

gtg tac gca gca ggt gac tgt act gac cta ttc cca ctg gcg tcc gtt        1975
Val Tyr Ala Ala Gly Asp Cys Thr Asp Leu Phe Pro Leu Ala Ser Val
310                 315                 320                 325

gca gcg atg cag ggc cgt atc gcc atg tat cac gca ctc ggt gaa ggc        2023
Ala Ala Met Gln Gly Arg Ile Ala Met Tyr His Ala Leu Gly Glu Gly
                330                 335                 340

gtg agc ccc atc cgt ttg aag act gtt gcc acc gca gtg ttt acc cgc        2071
Val Ser Pro Ile Arg Leu Lys Thr Val Ala Thr Ala Val Phe Thr Arg
            345                 350                 355

cca gag atc gca gca gta ggt atc acc cat gca caa gtt gat tcc ggc        2119
Pro Glu Ile Ala Ala Val Gly Ile Thr His Ala Gln Val Asp Ser Gly
        360                 365                 370

gaa gtg tct gct cgc gtg att gtg ctt cct ttg gct act aac cca cgc        2167
Glu Val Ser Ala Arg Val Ile Val Leu Pro Leu Ala Thr Asn Pro Arg
    375                 380                 385

gcc aag atg cgt tcc ctg cgc cac ggt ttt gtg aag ctg ttc tgc cgc        2215
Ala Lys Met Arg Ser Leu Arg His Gly Phe Val Lys Leu Phe Cys Arg
390                 395                 400                 405

cgt aac tct ggc ctg atc atc ggt ggt gtc gtg gtg gca ccg acc gcg        2263
Arg Asn Ser Gly Leu Ile Ile Gly Gly Val Val Val Ala Pro Thr Ala
                410                 415                 420

tct gag ctg atc cta ccg atc gct gtg gca gtg acc aac cgt ctg aca        2311
Ser Glu Leu Ile Leu Pro Ile Ala Val Ala Val Thr Asn Arg Leu Thr
            425                 430                 435

gtt gct gat ctg gct gat acc ttc gcg gtg tac cca tca ttg tca ggt        2359
Val Ala Asp Leu Ala Asp Thr Phe Ala Val Tyr Pro Ser Leu Ser Gly
        440                 445                 450

tcg att act gaa gca gca cgt cag ctg gtt caa cat gat gat cta ggc        2407
Ser Ile Thr Glu Ala Ala Arg Gln Leu Val Gln His Asp Asp Leu Gly
    455                 460                 465

taa tttttctgag tcttagattt tgagaaaacc caggattgct ttgtgcactc        2460

ctgggttttc actttgttaa gcagttttgg ggaaaagtgc aaagtttgca aagtttagaa        2520

atattttaag aggtaagatg tctgcaggtg gaagcgttta aatgcgttaa acttggccaa        2580

atgtggcaac ctttgcaagg tgaaaaactg gggcggggtt agatcctggg gggtttattt        2640

cattcacttt ggcttgaagt cgtgcaggtc aggggagtgt tgcccgaaaa cattgagagg        2700

aaaacaaaaa ccgatgtttg attgggggaa tcgggggtta cgatactagg acgcagtgac        2760

tgctatcacc cttggcggtc tcttgttgaa aggaataatt actctagtgt cgactcacac        2820

atcttcaacg cttccagcat tcaaaaagat cttggtagca aaccgcggcg aaatcgcggt        2880

ccgtgctttc cgtgcagcac tcgaaaccgg tgcagccacg gtagctattt acccccgtga        2940

agatcgggga tcattccacc gctcttttgc ttctgaagct gtccgcattg gtaccgaagg 3000

ctcaccagtc aaggcgtacc tggacatcga tgaaattatc ggtgcagcta aaaaagttaa 3060

agcagatgcc atttacccgg gatacggctt cctgtctgaa aatgcccagc ttgcccgcga 3120

gtgtgcggaa aacggcatta cttttattgg cccaacccca gaggttcttg atctcaccgg 3180

tgataagtct cgcgcggtaa ccgccgcgaa gaaggctggt ctgccagttt tggcggaatc 3240

caccccgagc aaaaacatcg atgagatcgt taaaagcgct gaaggccaga cttaccccat 3300

ctttgtgaag gcagttgccg gtggtggcgg acgcggtatg cgttttgttg cttcacctga 3360

tgagcttcgc aaattagcaa cagaagcatc tcgtgaagct gaagcggctt 3410

<210> 2
<211> 469
<212> PRT
<213> Corynebacterium glutamicum

<400> 2

Met Ala Lys Arg Ile Val Ile Ile Gly Gly Gly Pro Ala Gly Tyr Glu
1               5                   10                  15

Ala Ala Leu Ala Gly Ala Lys Tyr Gly Ala Glu Val Thr Val Ile Glu
            20                  25                  30

Asp Val Gly Val Gly Gly Ser Ala Val Thr Met Asp Cys Val Pro Ser
        35                  40                  45

Lys Ser Phe Ile Ala Gly Thr Gly Ile Lys Thr Asp Leu Arg Arg Ala
    50                  55                  60

Asp Asp Met Gly Leu Asn Arg Gly Leu Gly Lys Ala His Leu Glu Ile
65                  70                  75                  80

Asp Ala Leu Asn Ile Arg Val Lys Asp Leu Ala Lys Ala Gln Ser Glu
                85                  90                  95

Asp Ile Leu Gly Gln Leu Gln Arg Ser Asp Val Arg Met Ile Asn Gly
            100                 105                 110

Val Gly Arg Phe Asp Asp Tyr Asn Thr Lys Gln Thr Thr His Tyr Ile
        115                 120                 125

Lys Val Thr His Ser Asp Gly Ser Glu Glu Thr Val Glu Cys Asp Leu
    130                 135                 140

Val Leu Val Ala Thr Gly Ala Thr Pro Arg Ile Leu Lys Gly Ala Glu
145                 150                 155                 160

Pro Asp Gly Glu Arg Ile Leu Thr Trp Arg Gln Val Tyr Asp Ile Glu

                165                 170                 175

Glu Leu Pro Thr His Leu Ile Val Val Gly Ser Gly Val Thr Gly Ala
            180                 185                 190

Glu Phe Val Ser Ala Phe Ala Glu Leu Gly Val Lys Val Thr Met Val
        195                 200                 205

Ala Ser Arg Asp Arg Ile Leu Pro His Asp Asp Ala Asp Ala Ala Asp
    210                 215                 220

Val Leu Glu Thr Val Leu Ala Glu Arg Gly Val Ser Leu Glu Lys His
225                 230                 235                 240

Ala Arg Val Glu Ser Val Thr Arg Thr Glu Asp Gly Gly Val Cys Val
                245                 250                 255

Arg Thr Ala Asp Gly Arg Glu Ile Tyr Gly Ser His Ala Leu Met Thr
            260                 265                 270

Val Gly Ser Ile Pro Asn Thr Ala Asp Leu Gly Leu Glu Asn Ile Gly
        275                 280                 285

Val Glu Leu Ala Pro Ser Gly His Ile Lys Val Asp Arg Val Ser Arg
    290                 295                 300

Thr Asn Ile Pro Gly Val Tyr Ala Ala Gly Asp Cys Thr Asp Leu Phe
305                 310                 315                 320

Pro Leu Ala Ser Val Ala Ala Met Gln Gly Arg Ile Ala Met Tyr His
                325                 330                 335

Ala Leu Gly Glu Gly Val Ser Pro Ile Arg Leu Lys Thr Val Ala Thr
            340                 345                 350

Ala Val Phe Thr Arg Pro Glu Ile Ala Ala Val Gly Ile Thr His Ala
        355                 360                 365

Gln Val Asp Ser Gly Glu Val Ser Ala Arg Val Ile Val Leu Pro Leu
    370                 375                 380

Ala Thr Asn Pro Arg Ala Lys Met Arg Ser Leu Arg His Gly Phe Val
385                 390                 395                 400

Lys Leu Phe Cys Arg Arg Asn Ser Gly Leu Ile Ile Gly Gly Val Val
                405                 410                 415

Val Ala Pro Thr Ala Ser Glu Leu Ile Leu Pro Ile Ala Val Ala Val
            420                 425                 430

Thr Asn Arg Leu Thr Val Ala Asp Leu Ala Asp Thr Phe Ala Val Tyr
        435                 440                 445

Pro Ser Leu Ser Gly Ser Ile Thr Glu Ala Ala Arg Gln Leu Val Gln
    450                 455                 460

His Asp Asp Leu Gly
465

<210>  3
<211>  1706
<212>  DNA
<213>  Corynebacterium glutamicum

<220>
<221>  promoter
<222>  (612)..(1100)
<223>  Promoter Pg3 with RBS

<220>
<221>  gene
<222>  (1101)..(1700)
<223>  part of lpdA gene

<400>  3
cccggggatc catgcgccca acgcgatccc agattccggc atcgtgtcag gcaccttgcg      60

tacagccgac atctctacct ggcgtgacat gcgtccgctt atctctgagc tggtggaaca     120

ggtgctcgca cccaccggag tcacccatga actgatctac aatccgggtg ttccaccagt     180

gcttaacgac gatgtcgcca ccgctttgtt ggcaagcgca gcacgcgaca tggacacaca     240

atctgttgtc caagcgccgc agtcatccgg tggagaagac ttctcgtggt accttgaaca     300

cgtcccagga tcaatggccc ggttgggttg ctggccgggg cacggaccca agcaagacct     360

ccatcaaagt gacctggttg tggatgagcg agccatcgga gttggcgtca ggctctttgg     420

ctcccttgtg cagcagtaca gtagccgatc tgaagctttc ttaaattcct aatgggggta     480

gtgtgtaggg ctggctctaa attgctgaca cgtcacccag gctaattcag cagtaatcat     540

ttagacttgg aaccgcttac cagtggtttc aacaatgcat tcacccagct cacacgtgtg     600

gaggtgcctt aagtacttga agcctaaaaa cgaccgagcc tattgggatt accattgaag     660

ccagtgtgag ttgcatcaca ttggcttcaa atctgagact ttaatttgtg gattcacggg     720

ggtgtaatgt agttcataat taaccccatt cgggggagca gatcgtagtg cgaacgattt     780

caggttcgtt ccctgcaaaa actatttagc gcaagtgttg gaaatgcccc cgtttggggt     840

caatgtccat ttttgaatgt gtctgtatga ttttgcatct gctgcgaaat ctttgtttcc     900

ccgctaaagt tgaggacagg ttgacacgga gttgactcga cgaattatcc aatgtgagta     960

ggtttggtgc gtgagttgaa aaaattcgcc atactcgccc ttgggttctg tcagctcaag    1020

aattcttgag tgaccgatgc tctgattgac ctaactgctt gacacattgc atttcctaca 1080

atcgcgagag gagacacaac atggcaaaga ggatcgtaat tatcggcggt ggacctgcag 1140

gctatgaagc cgcactcgca ggcgctaaat acggtgcaga agttaccgtt attgaagatg 1200

tcggagttgg cggatccgca gtcaccatgg actgtgtacc ttcaaagtcc ttcatcgctg 1260

gtaccggtat caaaaccgac ctccgacgtg ctgatgacat gggacttaac cgtgggcttg 1320

gaaaagcaca cctagaaatc gatgcactga acatccgtgt gaaggacctt gcgaaagcac 1380

agtccgaaga tatcttgggc cagctgcagc gctcagatgt ccgcatgatt aacggtgtgg 1440

gccgctttga tgattacaac accaagcaaa ccacccacta cattaaagtc acccacagcg 1500

atggctccga agaaaccgtt gagtgcgatc tggtgctggt tgcaactggt gcaacccccc 1560

gcattcttaa aggtgcagag ccagacggcg agcgcatctt gacctggcgt caggtctacg 1620

acattgaaga actccccacc caccttatcg tggttggttc cggtgtgacc ggtgcggaat 1680

ttgtctctgc gtttgctgaa tctaga 1706

<210> 4
<211> 46
<212> DNA
<213> Corynebacterium glutamicum

<220>
<221> misc_feature
<222> (1)..(46)
<223> Primer lpdA_pZ8-1-1

<400> 4
ggtggtgaat tcaaaggagg acaaccatgg caaagaggat cgtaat 46

<210> 5
<211> 32
<212> DNA
<213> Corynebacterium glutamicum

<220>
<221> misc_feature
<222> (1)..(32)
<223> Primer lpdA_pZ8-1-2

<400> 5
ggtggtgtcg acttagccta gatcatcatg tt 32

<210> 6
<211> 1448
<212> DNA
<213> Corynebacterium glutamicum

<220>
<221> CDS
<222> (27)..(1436)
<223> lpdA gene

<400> 6
ggtggtgaat tcaaaggagg acaacc atg gca aag agg atc gta att atc ggc 53
Met Ala Lys Arg Ile Val Ile Ile Gly
1               5

ggt gga cct gca ggc tat gaa gcc gca ctc gca ggc gct aaa tac ggt 101
Gly Gly Pro Ala Gly Tyr Glu Ala Ala Leu Ala Gly Ala Lys Tyr Gly
10                  15                  20                  25

gca gaa gtt acc gtt att gaa gat gtc gga gtt ggc gga tcc gca gtc 149
Ala Glu Val Thr Val Ile Glu Asp Val Gly Val Gly Gly Ser Ala Val
                30                  35                  40

acc atg gac tgt gta cct tca aag tcc ttc atc gct ggt acc ggt atc 197
Thr Met Asp Cys Val Pro Ser Lys Ser Phe Ile Ala Gly Thr Gly Ile
            45                  50                  55

aaa acc gac ctc cga cgt gct gat gac atg gga ctt aac cgt ggg ctt 245
Lys Thr Asp Leu Arg Arg Ala Asp Asp Met Gly Leu Asn Arg Gly Leu
        60                  65                  70

gga aaa gca cac cta gaa atc gat gca ctg aac atc cgt gtg aag gac 293
Gly Lys Ala His Leu Glu Ile Asp Ala Leu Asn Ile Arg Val Lys Asp
    75                  80                  85

ctt gcg aaa gca cag tcc gaa gat atc ttg ggc cag ctg cag cgc tca 341
Leu Ala Lys Ala Gln Ser Glu Asp Ile Leu Gly Gln Leu Gln Arg Ser
90                  95                  100                 105

gat gtc cgc atg att aac ggt gtg ggc cgc ttt gat gat tac aac acc 389
Asp Val Arg Met Ile Asn Gly Val Gly Arg Phe Asp Asp Tyr Asn Thr
                110                 115                 120

aag caa acc acc cac tac att aaa gtc acc cac agc gat ggc tcc gaa 437
Lys Gln Thr Thr His Tyr Ile Lys Val Thr His Ser Asp Gly Ser Glu
            125                 130                 135

gaa acc gtt gag tgc gat ctg gtg ctg gtt gca act ggt gca acc ccc 485
Glu Thr Val Glu Cys Asp Leu Val Leu Val Ala Thr Gly Ala Thr Pro
        140                 145                 150

cgc att ctt aaa ggt gca gag cca gac ggc gag cgc atc ttg acc tgg 533
Arg Ile Leu Lys Gly Ala Glu Pro Asp Gly Glu Arg Ile Leu Thr Trp
    155                 160                 165

cgt cag gtc tac gac att gaa gaa ctc ccc acc cac ctt atc gtg gtt 581
Arg Gln Val Tyr Asp Ile Glu Glu Leu Pro Thr His Leu Ile Val Val
170                 175                 180                 185

ggt tcc ggt gtg acc ggt gcg gaa ttt gtc tct gcg ttt gct gaa ctc 629
Gly Ser Gly Val Thr Gly Ala Glu Phe Val Ser Ala Phe Ala Glu Leu
                190                 195                 200

ggc gtc aaa gtc acc atg gtg gca tcc cgt gac cgc att ttg cct cac 677
Gly Val Lys Val Thr Met Val Ala Ser Arg Asp Arg Ile Leu Pro His
            205                 210                 215

gat gac gca gat gcc gca gac gtg ctg gaa acc gtt ctg gct gag cgc 725
Asp Asp Ala Asp Ala Ala Asp Val Leu Glu Thr Val Leu Ala Glu Arg
        220                 225                 230

gga gta tcc ctg gaa aag cat gcc cgc gtg gag tct gtc acc cgc acc 773
Gly Val Ser Leu Glu Lys His Ala Arg Val Glu Ser Val Thr Arg Thr
    235                 240                 245

gaa gac ggt ggc gtg tgt gtt cgc act gct gac gga cga gaa atc tac          821
Glu Asp Gly Gly Val Cys Val Arg Thr Ala Asp Gly Arg Glu Ile Tyr
250                 255                 260                 265

ggt tct cac gcg ttg atg act gtt ggt tcc att cca aac acg gca gat          869
Gly Ser His Ala Leu Met Thr Val Gly Ser Ile Pro Asn Thr Ala Asp
                270                 275                 280

ctt ggc ctg gag aac atc ggt gtt gag ctg gca cca tcc ggc cat atc          917
Leu Gly Leu Glu Asn Ile Gly Val Glu Leu Ala Pro Ser Gly His Ile
            285                 290                 295

aag gtt gac cgc gtc tcc cgc acc aac atc ccc ggt gtg tac gca gca          965
Lys Val Asp Arg Val Ser Arg Thr Asn Ile Pro Gly Val Tyr Ala Ala
        300                 305                 310

ggt gac tgt act gac cta ttc cca ctg gcg tcc gtt gca gcg atg cag         1013
Gly Asp Cys Thr Asp Leu Phe Pro Leu Ala Ser Val Ala Ala Met Gln
    315                 320                 325

ggc cgt atc gcc atg tat cac gca ctc ggt gaa ggc gtg agc ccc atc         1061
Gly Arg Ile Ala Met Tyr His Ala Leu Gly Glu Gly Val Ser Pro Ile
330                 335                 340                 345

cgt ttg aag act gtt gcc acc gca gtg ttt acc cgc cca gag atc gca         1109
Arg Leu Lys Thr Val Ala Thr Ala Val Phe Thr Arg Pro Glu Ile Ala
                350                 355                 360

gca gta ggt atc acc cat gca caa gtt gat tcc ggc gaa gtg tct gct         1157
Ala Val Gly Ile Thr His Ala Gln Val Asp Ser Gly Glu Val Ser Ala
            365                 370                 375

cgc gtg att gtg ctt cct ttg gct act aac cca cgc gcc aag atg cgt         1205
Arg Val Ile Val Leu Pro Leu Ala Thr Asn Pro Arg Ala Lys Met Arg
        380                 385                 390

tcc ctg cgc cac ggt ttt gtg aag ctg ttc tgc cgc cgt aac tct ggc         1253
Ser Leu Arg His Gly Phe Val Lys Leu Phe Cys Arg Arg Asn Ser Gly
    395                 400                 405

ctg atc atc ggt ggt gtc gtg gtg gca ccg acc gcg tct gag ctg atc         1301
Leu Ile Ile Gly Gly Val Val Val Ala Pro Thr Ala Ser Glu Leu Ile
410                 415                 420                 425

cta ccg atc gct gtg gca gtg acc aac cgt ctg aca gtt gct gat ctg         1349
Leu Pro Ile Ala Val Ala Val Thr Asn Arg Leu Thr Val Ala Asp Leu
                430                 435                 440

gct gat acc ttc gcg gtg tac cca tca ttg tca ggt tcg att act gaa         1397
Ala Asp Thr Phe Ala Val Tyr Pro Ser Leu Ser Gly Ser Ile Thr Glu
            445                 450                 455

gca gca cgt cag ctg gtt caa cat gat gat cta ggc taa gtcgacacca cc       1448
Ala Ala Arg Gln Leu Val Gln His Asp Asp Leu Gly
        460                 465

<210>  7
<211>  469
<212>  PRT
<213>  Corynebacterium glutamicum

<400>  7

Met Ala Lys Arg Ile Val Ile Ile Gly Gly Gly Pro Ala Gly Tyr Glu
1               5                   10                  15

Ala Ala Leu Ala Gly Ala Lys Tyr Gly Ala Glu Val Thr Val Ile Glu
            20                  25                  30

Asp Val Gly Val Gly Gly Ser Ala Val Thr Met Asp Cys Val Pro Ser
        35                  40                  45

Lys Ser Phe Ile Ala Gly Thr Gly Ile Lys Thr Asp Leu Arg Arg Ala
    50                  55                  60

Asp Asp Met Gly Leu Asn Arg Gly Leu Gly Lys Ala His Leu Glu Ile
65                  70                  75                  80

Asp Ala Leu Asn Ile Arg Val Lys Asp Leu Ala Lys Ala Gln Ser Glu
                85                  90                  95

Asp Ile Leu Gly Gln Leu Gln Arg Ser Asp Val Arg Met Ile Asn Gly
            100                 105                 110

Val Gly Arg Phe Asp Asp Tyr Asn Thr Lys Gln Thr Thr His Tyr Ile
        115                 120                 125

Lys Val Thr His Ser Asp Gly Ser Glu Glu Thr Val Glu Cys Asp Leu
    130                 135                 140

Val Leu Val Ala Thr Gly Ala Thr Pro Arg Ile Leu Lys Gly Ala Glu
145                 150                 155                 160

Pro Asp Gly Glu Arg Ile Leu Thr Trp Arg Gln Val Tyr Asp Ile Glu
                165                 170                 175

Glu Leu Pro Thr His Leu Ile Val Val Gly Ser Gly Val Thr Gly Ala
            180                 185                 190

Glu Phe Val Ser Ala Phe Ala Glu Leu Gly Val Lys Val Thr Met Val
        195                 200                 205

Ala Ser Arg Asp Arg Ile Leu Pro His Asp Asp Ala Asp Ala Ala Asp
    210                 215                 220

Val Leu Glu Thr Val Leu Ala Glu Arg Gly Val Ser Leu Glu Lys His
225                 230                 235                 240

Ala Arg Val Glu Ser Val Thr Arg Thr Glu Asp Gly Gly Val Cys Val
                245                 250                 255

Arg Thr Ala Asp Gly Arg Glu Ile Tyr Gly Ser His Ala Leu Met Thr

260                 265                 270

Val Gly Ser Ile Pro Asn Thr Ala Asp Leu Gly Leu Glu Asn Ile Gly
        275                 280                 285

Val Glu Leu Ala Pro Ser Gly His Ile Lys Val Asp Arg Val Ser Arg
    290                 295                 300

Thr Asn Ile Pro Gly Val Tyr Ala Ala Gly Asp Cys Thr Asp Leu Phe
305                 310                 315                 320

Pro Leu Ala Ser Val Ala Ala Met Gln Gly Arg Ile Ala Met Tyr His
                325                 330                 335

Ala Leu Gly Glu Gly Val Ser Pro Ile Arg Leu Lys Thr Val Ala Thr
            340                 345                 350

Ala Val Phe Thr Arg Pro Glu Ile Ala Ala Val Gly Ile Thr His Ala
        355                 360                 365

Gln Val Asp Ser Gly Glu Val Ser Ala Arg Val Ile Val Leu Pro Leu
    370                 375                 380

Ala Thr Asn Pro Arg Ala Lys Met Arg Ser Leu Arg His Gly Phe Val
385                 390                 395                 400

Lys Leu Phe Cys Arg Arg Asn Ser Gly Leu Ile Ile Gly Gly Val Val
                405                 410                 415

Val Ala Pro Thr Ala Ser Glu Leu Ile Leu Pro Ile Ala Val Ala Val
            420                 425                 430

Thr Asn Arg Leu Thr Val Ala Asp Leu Ala Asp Thr Phe Ala Val Tyr
        435                 440                 445

Pro Ser Leu Ser Gly Ser Ile Thr Glu Ala Ala Arg Gln Leu Val Gln
    450                 455                 460

His Asp Asp Leu Gly
465

<210>  8
<211>  20
<212>  DNA
<213>  Corynebacterium glutamicum

<400>  8
aacacgtccc aggatcaatg                                                20

<210>  9

<211>  20
<212>  DNA
<213>  Corynebacterium glutamicum

<400>  9
tttcgcaagg tccttcacac                                                    20

12
201100472

92

## Beschreibung

Die Erfindung betrifft einen Mikroorganismus, der eine organisch-chemische Verbindung produziert und/oder ausscheidet, wobei der Mikroorganismus eine erhöhte Aktivität einer Transhydrogenase (LpdA Protein, Genprodukt des lpdA Gens) aufweist; sowie ein Verfahren zur Herstellung einer organisch-chemischen Verbindung unter Verwendung des erfindungsgemäßen Mikroorganismus.

Die vorliegende Erfindung ist im Rahmen eines vom Bundesministerium für Bildung und Forschung (BMBF) geförderten Projektes (Förderkennzeichen 0313917A) zustandegekommen.

### Stand der Technik

Organisch-chemische Verbindungen, zu denen insbesondere Aminosäuren, organische Säuren, Vitamine, Nukleoside und Nukleotide zählen, finden in der Humanmedizin, in der pharmazeutischen Industrie, in der Lebensmittelindustrie und ganz besonders in der Tierernährung Anwendung.

Zahlreiche dieser Verbindungen werden, wie beispielsweise das L-Lysin, durch Fermentation von Stämmen coryneformer Bakterien, insbesondere Corynebacterium glutamicum, oder von Stämmen der Familie der Enterobacteriaceae, insbesondere Escherichia coli, hergestellt. Wegen der großen ökonomischen Bedeutung wird ständig an der Verbesserung der Herstellverfahren gearbeitet. Verfahrensverbesserungen können fermentationstechnische Maßnahmen wie zum Beispiel Rührung und Versorgung mit Sauerstoff, oder die Zusammensetzung der Nährmedien, wie zum Beispiel die Zuckerkonzentration während der Fermentation, oder die Aufarbeitung zur Produktform durch zum Beispiel Ionenaustauschchromatographie oder die intrinsischen Leistungseigenschaften des Mikroorganismus selbst betreffen.

Biosynthesewege von Aminosäuren unterliegen in Wildtyp-Stämmen einer strengen metabolischen Kontrolle, die gewährleistet, dass die Aminosäuren nur in dem für den Eigenbedarf der Zelle erforderlichen Maß hergestellt werden. Eine wichtige Voraussetzung für effiziente Produktionsprozesse ist es deshalb, dass geeignete Mikroorganismen verfügbar sind, die im Gegensatz zu Wildtyp-Organismen eine drastisch über den Eigenbedarf hinaus gesteigerte Produktionsleistung (Überproduktion) für die Herstellung der gewünschten Aminosäure aufweisen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite oder auxotroph für regulatorisch bedeutsame Metabolite sind und die L-Aminosäuren produzieren. Ein bekannter Antimetabolit ist das Lysinanalogon S-(2-Aminoethyl)-L-Cystein (AEC).

Seit einigen Jahren werden ebenfalls Methoden der rekombinanten DNA-Technik zur Stammverbesserung von L-Aminosäure produzierenden Stämmen der Gattung Corynebacterium, insbesondere Corynebacterium glutamicum, eingesetzt, oder der Gattung Escherichia, insbesondere Escherichia coli, indem man einzelne Aminosäure-Biosynthesegene modifiziert bzw. verstärkt oder abschwächt und die Auswirkung auf die Aminosäure-Produktion untersucht.

Die Nukleotidsequenzen der Chromosomen zahlreicher Bakterien sind bekannt.

Die Nukleotidsequenz des Genoms von Corynebacterium glutamicum ATCC13032 ist bei Ikeda und Nakagawa (Applied Microbiology and Biotechnology 62, 99-109 (2003)), in der EP 1 108 790 und bei Kalinowski et al. (Journal of Biotechnolgy 104(1-3), (2003)) beschrieben.

Die Nukleotidsequenz des Genoms von Corynebacterium glutamicum R ist bei Yukawa et al. (Microbiology 153(4):1042-1058 (2007)) beschrieben.

Die Nukleotidsequenz des Genoms von Corynebacterium efficiens ist bei Nishio et al (Genome Research. 13 (7), 1572-1579 (2003)) beschrieben.

Die Nukleotidsequenz des Genoms von Corynebacterium diphteriae NCTC 13129 wurde von Cerdeno-Tarraga et al. (Nucleic Acids Research 31 (22), 6516-6523 (2003)) beschrieben.

Die Nukleotidsequenz des Genoms von Corynebacterium jeikeium wurde von Tauch et al. (Journal of Bacteriology 187 (13), 4671-4682 (2005)) beschrieben.

Die Nukleotidsequenzen des Genoms von Corynebacterium glutamicum sind ebenfalls in der Datenbank des National Center for Biotechnology Information (NCBI) der National Library of Medicine (Bethesda, MD, USA), in der DNA Data Bank of Japan (DDBJ, Mishima, Japan) oder in der Nukleotidsequenz-Datenbank der European Molecular Biologies Laboratories (EMBL, Heidelberg, Deutschland bzw. Cambridge, UK) verfügbar.

Eine zusammenfassende Darstellung zu verschiedenen Aspekten der fermentativen Produktion von L-Aminosäuren findet man bei R. Faurie und J. Thommel in Advances in Biochemical Engineering Biotechnology, Band 79 (Springer-Verlag, Berlin, Heidelberg (Deutschland) 2003).

Bei der Herstellung der vorstehend genannten organisch-chemischen Verbindungen unter Einsatz von Mikroorganismen wird jede dieser Verbindungen in einem Biosyntheseweg in der Zelle eines Mikroorganismus hergestellt. Eines der wichtigen Coenzyme, die für die Funktion von wesentlichen Enzymen in dem Biosynthesesystem essentiell sind, ist reduziertes Nicotinamidadenindinukleotidphosphat (im Folgenden als NADPH bezeichnet).

Die Beziehung zwischen NADPH und der Herstellung von organisch-chemischen Verbindungen unter Einsatz von Mikroorganismen wird zum Beispiel in der EP0733712B erläutert.

Nicotinamiddinukleotidtranshydrogenase (im Folgenden einfach als "Transhydrogenase" bezeichnet) ist als eines der Enzyme bekannt, die für die Produktion von NADPH verantwortlich sind. Es ist bekannt, dass dieses Enzym in verschiedenen Organismen vorhanden ist, einschließlich in Mikroorganismen der Gattung Escherichia. In Escherichia coli, ein typischer Mikroorganismus der Gattung Escherichia, wurde die Reinigung von Transhydrogenase (David M. Clarke and Philip D. Bragg, Eur. J. Biochem., 149, 517-523 (1985)), das Klonieren eines dafür kodierenden Gens (David M. Clarke and Philip D. Bragg, J. Bacteriology., 162, 367-373 (1985)) und die Bestimmung der Nukleotidsequenz des Gens (David M. Clarke, Tip W. Loo, Shirley Gillam, and Philip D. Bragg, Eur. J. Biochem. 158, 647-653 (1986)) durchgeführt, und es wurde das Vorhandensein des Enzyms gezeigt. Eine physiologische Funktion des Enzyms ist bislang jedoch fast unbekannt. Dies wird typischerweise durch die Tatsache gezeigt, dass Varianten, denen das Enzym fehlt, keine phänotypische Expression zeigen.

Makoto Ishimoto"Metabolic Maps, July 25, 1971 (Kyoritsü Suppan Co., Ltd.), Seiten'30-32, offenbart, dass die reduzierende Aktivität von NADH oder NADPH für die Biosynthese mehrerer Aminosäuren erforderlich ist. Dieses Dokument offenbart jedoch nicht die Umwandlung von NADH in NADPH für die Biosynthese einer Zielsubstanz.

Bunji Maruo, Nobuo Tamiya (Autoren) "Enzyme Handbook", March 1, 1983 (01.03.83), Asakura Shoten), S. 132-133 und Arch. Biochem. Biophys. Vol. 176, Nr. 1, 1976, Wermuth B et al. "Pyridine nucleotide transhydrogenase from pseudomonas aeruginosa purification by affinity chromatography and physiochemical properties", S. 136-143, offenbaren jeweils ein Enzym, das in der Lage ist, NADH in NADPH und umgekehrt umzuwandeln. Keines dieser Dokumente betrifft die Herstellung einer Zielsubstanz oder eine erhöhte Produktivität für NADPH, die für die Herstellung einer Zielsubstanz eingesetzt werden könnte.

E. coli enthält sowohl eine lösliche als auch eine membrangebundene Pyridin-Nukleotid-Transhydrogenase. Die lösliche Pyridin-Nukleotid-Transhydrogenase ist das Genprodukt SthA (Sth, UdhA); seine primäre physiologische Rolle scheint der Reoxidation von NADPH. Die membrangebundene Protonen-übertragende Pyridin-Nukleotid-Transhydrogenase ist das PntAB Genprodukt; PntAB ist eine wichtige Quelle von NADPH (Sauer et al., The Journal of Biological Chemistry 279(8) (2004)).

Anderlund et al. (Applied and Environmental Microbiology 56(6) (1999)) untersuchten die physiologische Wirkung der Expression der Membran-gebundenen Transhydrogenase (pntA- und pntB-Gene) aus Escherichia coli in rekombinanten Saccharomyces cerevisiae.

Vor diesem Hintergrund besteht die der vorliegenden Erfindung zu Grunde liegende Aufgabe darin, einen gegenüber den im Stand der Technik beschriebenen Mikroorganismen verbesserten Mikroorganismus, Verfahren zur Herstellung eines solchen Mikroorganismus und Verfahren unter Verwendung eines solchen Mikroorganismus zur Überproduktion von organisch chemischen Verbindungen bereitzustellen, wobei die Verbesserung Faktoren wie die intrazellulär erreichte Konzentration der überproduzierten Verbindung, die Ausbeute der überproduzierten Verbindung nach Aufbereitung der Kultur, die Wachstumseigenschaften des Mikroorganismus sowie die für die Überproduktion und Aufbereitung der Verbindung benötigte Zeit und Zahl von Verfahrensschritten sowie den Ressourcenbedarf des Prozesses, beispielsweise mit Hinblick auf Zeit, Energie und Menge der eingesetzten Stämme und Edukte, betrifft.

Diese und weitere Aufgaben werden durch den Gegenstand der vorliegenden Anmeldung und insbesondere durch den Gegenstand der unabhängigen Ansprüche gelöst, wobei sich Ausführungsformen aus den Unteransprüchen ergeben.

In einem ersten Aspekt wird die der Anmeldung zugrunde liegende Aufgabe gelöst durch ein Verfahren zur fermentativen Herstellung einer organisch-chemischen Verbindung unter Verwendung eines Mikroorganismus enthaltend die Schritte:
a. Fermentation eines eine organisch-chemische Verbindung produzierenden Mikroorganismus,
   wobei in dem Mikroorganismus ein Polynukleotid, das für ein Polypeptid kodiert dessen Aminosäuresequenz wenigstens 80%, wenigstens 85%, wenigstens 90%, wenigstens 95%, wenigstens 98% oder wenigstens 99% identisch ist mit der Aminosäuresequenz von SEQ ID NO:2, überexprimiert vorliegt und wobei die Fermentation in einem geeigneten Fermentationsmedium unter Bildung einer Fermentationsbrühe abläuft und
b.Anreichern (Akkumulieren) der organisch-chemischen Verbindung in der Fermentationsbrühe aus a).

Im Rahmen der vorliegenden Erfindung ist unter einem eine organisch-chemische Verbindung produzierenden Mikroorganismus ein Mikroorganismus zu verstehen, der die organisch-chemische Verbindung über den Bedarf hinaus, der zur Erhaltung der Lebensfähigkeit des Mikroorganismus erforderlich ist, produziert.

Im Rahmen der vorliegenden Erfindung ist unter dem Anreichern, bzw. Akkumulieren der organisch-chemischen Verbindung in der Fermentationsbrühe sowohl die Anreicherung/Akkumulierung der organisch-chemischen Verbindungen in den Mikrorganismenzellen als auch die Ausscheidung der organisch-chemischen Verbindung in das den Mikroorganismus umgebende Nährmedium, d.h. in die Fermentationsbrühe, zu verstehen.

Die der Anmeldung zugrunde liegende Aufgabe wird in einem weiteren Aspekt gelöst durch einen Mikroorganismus, der eine organisch-chemische Verbindung produziert, wobei in dem Mikrorganismus ein ein Polynukleotid, das für ein Polypeptid kodiert dessen Aminosäuresequenz wenigstens 80%, wenigstens 85%, wenigstens 90%, wenigstens 95%, wenigstens 98% oder wenigstens 99% identisch ist mit der Aminosäuresequenz von SEQ ID NO:2, überexprimiert vorliegt.

In einer Ausführungsform des erfindungsgemäßen Verfahrens, zur Herstellung einer organisch-chemischen Verbindung, bzw. des erfindungsgemäßen Mikrorganismus weist das kodierte Polypeptid, dessen Aminosäuresequenz wenigstens 80%, wenigstens 85%, wenigstens 90%, wenigstens 95%, wenigstens 98% oder wenigstens 99% identisch ist mit der Aminosäuresequenz von SEQ ID NO:2, die Aktivität einer Transhydrogenase auf.

Die Aktivität einer Transhydrogenase wird über den Enzymtest nach Argyrou et al. (2004) nachgewiesen. Hierbei wird aufgereinigte Transhydrogenase hinsichtlich der NAD(P)H-abhängigen Reduktion von 5-HNQ, einem artifiziellen Elektronenakzeptor, untersucht.

Die für die Maßnahmen der Überexpression des anspruchsgemäß offenbarten Polypeptids eingesetzten Mikroorganismen bzw. Stämme (Ausgangsstämme) besitzen bevorzugt bereits die Fähigkeit die gewünschte organische chemische Verbindung in der Zelle anzureichern oder in das sie umgebende Nährmedium auszuscheiden und dort zu akkumulieren.

Der Vorteil der vorliegenden Erfindung liegt darin, dass die Produktausbeute bei biotechnologischen Produktionsprozessen, z.B. der Aminosäureproduktion, weiter erhöht wird. Mit dem erfindungsgemäßen Verfahren, bzw. mit der Verwendung der erfindungsgemäßen Mikroorganismen kann die mikrobielle Produktion von organisch-chemischen Verbindungen weiter gesteigert werden.

Daher zeichnet sich das erfindungsgemäße Verfahren zur Herstellung einer organisch-chemischen Verbindung auch dadurch aus, dass die Herstellung der organisch chemischen Verbindung gegenüber der Fermentation eines Mikroorganismus, in dem das Polynukleotid, das für ein Polypeptid kodiert dessen Aminosäuresequenz wenigstens 80% identisch ist mit der Aminosäuresequenz von SEQ ID NO:2, nicht überexprimiert vorliegt, um mindestens 0,5%, mindestens 1%, mindestens 1,5% oder mindestens 2% erhöht ist. Überraschenderweise wurde über eine hohe Sequenzidentität zu einem Protein mit der Aktivität einer Transhydrogenase (LpdA Protein) in Mycobacterium tuberculosis eine Transhydrogenase in C. glutamicum identifiziert. Die Verstärkung der Expression der Transhydrogenase führte zu einer erhöhten Produktion einer organisch-chemischen Verbindung bei Verwendung eines entsprechenden Produktionsstammes.

In einer bevorzugten Ausführungsform des erfindungsgemäßen Verfahren/ des erfindungsgemäßen Mikroorganismus stammt das Polynukleotid, das für ein Polypeptid kodiert dessen Aminosäuresequenz wenigstens 80%, wenigstens 85%, wenigstens 90%, wenigstens 95%, wenigstens 98% oder wenigstens 99%, bzw.100% identisch ist mit der Aminosäuresequenz von SEQ ID NO:2 aus Corynebacterium glutamicum. Besonders bevorzugt weist dieses kodierte Polypeptid die Aktivität einer Transhydrogenase auf.

Der erfindungsgemäße Mikroorganismus weist eine im Vergleich zu dem jeweiligen Ausgangstamm erhöhte Expression eines Polynukleotids kodierend für ein Polypeptid mit einer Aminosäuresequenz, die eine Identität von 80% oder mehr zu der in SEQ ID NO:2 gezeigten Aminosäuresequenz zeigt, auf.

In bevorzugten Ausführungsformen sind Varianten umfasst, die wenigstens 80%, wenigstens 85%, wenigstens 90%, wenigstens 95%, wenigstens 98% oder wenigstens 99% identisch zu der in SEQ ID NO:2 gezeigten Aminosäuresequenz sind, d.h. wobei wenigstens 80%, wenigstens 85%, wenigstens 90%, wenigstens 95%, wenigstens 98% oder wenigstens 99% der Aminosäurepositionen identisch zu der in SEQ ID NO:2 gezeigten Aminosäuresequenz sind. Die prozentuale Identität wird vorzugsweise über die gesamte Länge der Aminosäure oder Nukleinsäureregion berechnet.

In einer bevorzugten Ausführungsform weist der Mikroorganismus eine im Vergleich zu dem jeweiligen Ausgangstamm erhöhte Expression eines Polynukleotids kodierend für ein Polypeptid mit einer Aminosäuresequenz gemäß SEQ ID NO:2 auf.

Da für die Biosynthese von organisch-chemischen Verbindungen Reduktionsäquivalente in Form von NADPH benötigt werden, ist die verbesserte Versorgung mit Reduktionsäquivalenten vorteilhaft.

Unter einer organisch-chemischen Verbindung versteht man für die Maßnahmen der Erfindung ein Vitamin wie z.B. Thiamin (Vitamin B1), Riboflavin (Vitamin B2), Cyanocobalamin (Vitamin B12), Folsäure (Vitamin M), Tocopherol (Vitamin E) oder Nicotinsäure/Nicotinsäureamid, ein Nukleosid oder Nukleotid wie z.B. S-Adenosyl-Methionin, Inosin-5-'-Monophosphorsäure und Guanosin-5'-Monophosphorsäure, L-Aminosäuren, organische Säuren oder auch ein Amin wie z.B. Cadaverin. Bevorzugt hergestellt werden L-Aminosäuren und diese enthaltende Produkte.

Die organisch-chemische Verbindung, die von dem erfindungsgemäßen Mikroorganismus produziert und/oder ausgeschieden wird, ist bevorzugt ausgewählt aus der Gruppe Vitamin, Nukleosid oder Nukleotid, L-Aminosäuren, organische Säuren und Amin.

Der Begriff L-Aminosäuren umfasst die proteinogenen Aminosäuren, sowie L-Ornithin und L-Homoserin. Unter proteinogenen L-Aminosäuren versteht man die L-Aminosäuren die in natürlichen Proteinen, das heißt in Proteinen von Mikroorganismen, Pflanzen, Tieren und Menschen, vorkommen. Zu den proteinogenen Aminosäuren zählen L-Asparaginsäure, L-Asparagin, L-Threonin, L-Serin, L-Glutaminsäure, L-Glutamin, L-Glycin, L-Alanin, L-Cystein, L-Valin, L-Methionin, L-Isoleucin, L-Leucin, L-Tyrosin, L-Phenylalanin, L-Histidin, L-Lysin, L-Tryptophan, L-Arginin, L-Prolin und gegebenenfalls L-Selenocystein und L-Pyrrolysin.

Der Begriff organische Säure umfasst γ-Ketosäuren, insbesondere eine α-Ketosäure ausgewählt aus der Gruppe α-Ketoisocapronsäure, α-Ketovaleriansäure und α-Keto-β-methylvaleriansäure.

In besonders bevorzugter Weise ist die organisch-chemische Verbindung ausgewählt aus der Gruppe proteinogene L-Aminosäure, L-Ornithin und L-Homoserin sowie α-Ketoisocapronsäure, α-Ketovaleriansäure und α-Keto-β-methylvaleriansäure. Besonders bevorzugt ist die proteinogene L-Aminosäure ausgewählt aus der Gruppe L-Threonin, L-Lysin, L-Methionin, L-Valin, L-Prolin, L-Glutamat, L-Leucin und L-Isoleucin, insbesondere L-Lysin, L-Methionin und L-Valin, ganz besonders L-Lysin und L-Methionin.

Werden im Folgenden Aminosäuren oder L-Aminosäuren erwähnt, umfasst der Begriff auch deren Salze wie beispielsweise das Lysin-Monohydrochlorid oder Lysin-Sulfat im Falle der Aminosäure L-Lysin. Ebenso umfasst der Begriff α-Ketosäure auch deren Salze wie im Fall der α-Ketoisocapronsäure (KIC) das Calcium-KIC, das Kalium-KIC oder das Natrium-KIC.

Der Mikroorganismus ist bevorzugt ausgewählt aus der Gruppe Bakterien, Hefe und Pilze, unter den Bakterien besonders bevorzugt aus der Familie Corynebacteriaceae oder der Familie Enterobacteriaceae, wobei die Gattung Corynebacterium oder die Gattung Escherichia ganz besonders bevorzugt und aus den genannten Gattungen die Art Corynebacterium glutamicum oder die Art Escherichia Coli besonders bevorzugt wird.

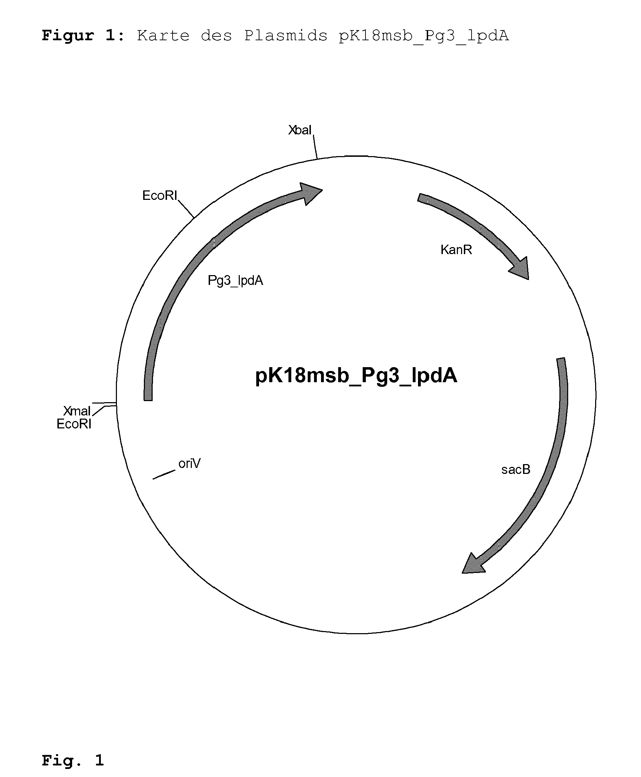

In einer weiteren bevorzugten Ausführungsform wird die Expression des Polynukleotids kodierend für ein Polypeptid dessen Aminosäuresequenz wenigstens 80%, wenigstens 85%, wenigstens 90%, wenigstens 95%, wenigstens 98% oder wenigstens 99%, bzw.100% identisch ist mit der Aminosäuresequenz von SEQ ID NO:2 und bevorzugt mit der Aktivität einer Transhydrogenase durch eine oder mehrere Maßnahmen ausgewählt aus der folgenden Gruppe erhöht:
a) die Expression des Gens steht unter der Kontrolle eines Promotors, der in dem für das Verfahren verwendeten Mikroorganismus stärker ist als der native, bzw. der ursprüngliche Promotor des Gens; Beispiele für erfindungsgemäß bevorzugt in Corynebacterium glutamicum einsetzbare Promotoren sind beispielsweise in Fig. 1 des Übersichtsartikels von Patek et al. (Journal of Biotechnology 104(1-3), 311-323 (2003)) beschrieben. In gleicher Weise können die von Vasicova et al. (Journal of Bacteriology 181, 6188-6191 (1999)) beschrieben Varianten des dapA-Promotors, beispielsweise der Promotor A25 eingesetzt werden. Weiterhin kann der gap-Promotor von Corynebacterium glutamicum (EP 06007373) verwendet werden. Schließlich können die hinlänglich bekannten von Amann et al. (Gene 69(2), 301-315 (1988)) und Amann und Brosius (Gene 40(2-3), 183-190 (1985)) beschriebenen Promotoren T3, T7, SP6, M13, lac, tac und trc verwendet werden.
b) Erhöhung der Kopienzahl des Gens kodierend für ein Polypeptid mit der Aktivität einer Transhydrogenase; vorzugsweise durch Einfügen des Gens in Plasmiden mit erhöhter Kopienzahl und/oder durch Integration des Gens in das Chromosom des Mikroorganismus in mindestens einer Kopie;
c) die Expression des Gens erfolgt unter Verwendung einer Ribosomen-Bindestelle, die in dem für das Verfahren verwendeten Mikroorganismus stärker ist als die ursprüngliche Ribosomen-Bindestelle des Gens;
d) die Expression des Gens erfolgt unter Optimierung der Kodon-Verwendung (codon usage) des für das Verfahren verwendeten Mikroorganismus;
e) die Expression des Gens erfolgt unter Reduzierung von mRNA Sekundärstrukturen in der vom Gen transkribierten mRNA;
f) die Expression des Gens erfolgt unter Eliminierung von RNA-Polymerase-Terminatoren in der vom Gen transkribierten mRNA;
g) die Expression des Gens erfolgt unter Verwendung mRNA-stabilisierender Sequenzen in der vom Gen transkribierten mRNA.

Die genannten Maßnahmen zur Erhöhung der Expression können in geeigneter Weise miteinander kombiniert werden. Bevorzugt wird die Expression des Polynukleotids kodierend für ein Polypeptidmit der Aktivität einer Transhydrogenase durch die Kombination mindestens zwei der Maßnahmen ausgewählt aus der Gruppe a), b) und c) erhöht, besonders bevorzugt durch die Kombination der Maßnahmen a) und b).

Wie oben erwähnt umfasst die vorliegende Erfindung einen Mikroorganismus sowie ein Verfahren zur fermentativen Herstellung einer organisch-chemischen Verbindung enthaltend die Schritte:
a) Kultivieren des oben beschriebenen Mikroorganismus gemäß der vorliegenden Erfindung in einem geeignetem Medium, wobei eine Fermentationsbrühe erhalten wird, und
b) Anreichern der organisch-chemischen Verbindung in der Fermentationsbrühe aus a).

Dabei ist bevorzugt, dass man aus der Fermentationsbrühe ein Produkt in flüssiger oder fester Form herstellt.

Wie oben erwähnt, umfasst der Begriff Mikroorganismus Bakterien, Hefen und Pilze. Unter den Bakterien sind insbesondere die Gattung Corynebacterium und die Gattung Escherichia zu nennen.

Innerhalb der Gattung Corynebacterium werden Stämme bevorzugt, die auf folgenden Arten beruhen:
Corynebacterium efficiens, wie zum Beispiel der Typstamm DSM44549,
Corynebacterium glutamicum, wie zum Beispiel der Typstamm ATCC13032 oder der Stamm R, und
Corynebacterium ammoniagenes, wie zum Beispiel der Stamm ATCC6871,
wobei die Stämme der Art Corynebacterium glutamicum ganz besonders bevorzugt wird.

Einige Stämme der Art Corynebacterium glutamicum sind im Stand der Technik auch unter anderen Bezeichnungen bekannt. Hierzu gehören beispielsweise:
Stamm ATCC13870 der als Corynebacterium acetoacidophilum bezeichnet wurde,
Stamm DSM20137 der als Corynebacterium lilium bezeichnet wurde,
Stamm ATCC17965 der als Corynebacterium melassecola bezeichnet wurde,
Stamm ATCC14067 der als Brevibacterium flavum bezeichnet wurde,
Stamm ATCC13869 der als Brevibacterium lactofermentum bezeichnet wurde, und
Stamm ATCC14020 der als Brevibacterium divaricatum bezeichnet wurde.

Der Begriff "Micrococcus glutamicus" für Corynebacterium glutamicum war ebenfalls gebräuchlich.

Einige Stämme der Art Corynebacterium efficiens wurden im Stand der Technik auch als Corynebacterium thermoaminogenes bezeichnet, wie zum Beispiel der Stamm FERM BP-1539.

Die Vertreter der Enterobacteriaceae werden bevorzugt ausgewählt aus den Gattungen Escherichia, Erwinia, Providencia und Serratia. Die Gattungen Escherichia und Serratia werden besonders bevorzugt. Bei der Gattung Escherichia ist insbesondere die Art Escherichia coli und bei der Gattung Serratia insbesondere die Art Serratia marcescens zu nennen.

Die für die Maßnahmen der Überexpression des anspruchsgemäß offenbarten Polypeptids eingesetzten Mikroorganismen bzw. Stämme (Ausgangsstämme) besitzen bevorzugt bereits die Fähigkeit die gewünschte(n) organisch-chemischen Verbindungen in der Zelle anzureichern oder in das sie umgebende Nährmedium auszuscheiden und dort zu akkumulieren. Hierfür wird im Folgenden auch der Ausdruck "produzieren" verwendet. Insbesondere besitzen die für die Maßnahmen der Überexpression eingesetzten Stämme die Fähigkein ≥ (mindestens) ≥ 0,10 g/l, 0,25 g/l, ≥ 0,5 g/l, ≥ 1,0 g/l, ≥ 1,5 g/l, ≥ 2,0 g/l, ≥ 4 g/l oder ≥ 10 g/l der gewünschten Verbindung in ≥ (maximal) 120 Stunden, ≤ 96 Stunden, ≤ 48 Stunden, ≤ 36 Stunden, ≤ 24 Stunden oder ≤ 12 Stunden in der Zelle oder im Nährmedium anzureichern bzw. zu akkumulieren. Bei den Ausgangsstämmen handelt es sich bevorzugt um Stämme, die durch Mutagenese und Selektion, durch rekombinante DNA-Techniken oder durch eine Kombination beider Methoden hergestellt wurden.

Es ist für den Fachmann verständlich, dass man zu einem für die Maßnahmen der Erfindung geeignetem Mikroorganismus auch dadurch gelangen kann, indem man in einem Wildstamm, wie zum Beispiel in dem Corynebacterium glutamicum Typstamm ATCC 13032 oder in dem Stamm ATCC 14067, zunächst eine Transhydrogenase überexprimiert und anschließend durch weitere, im Stand der Technik beschriebene, genetische Maßnahmen, den Mikrorganismus veranlasst, die gewünschte(n) L-Aminosäure(en) zu produzieren. Die Transformation des Wildtyps allein mit dem genannten Polynukleotid ist keine erfindungsgemäße Maßnahme.

L-Lysin ausscheidende bzw. produzierende Stämme der Art Corynebacterium glutamicum, die als Ausgangsstamm für die erfindungsgemäß die Transhydrogenase überexprimierenden Mikroorganismen dienen können, sind beispielsweise:
Corynebacterium glutamicum DSM13994 beschrieben in US 6,783,967, und
Corynebacterium glutamicum DM1933 beschrieben bei Blombach et al. (Appl Environ Microbiol. 2009 Jan;75(2):419-27).

Ein L-Lysin ausscheidender bzw. produzierender Stamm der Art Corynebacterium efficiens ist beispielsweise:
Corynebacterium thermoaminogenes AJ12521 (= FERM BP-3304) beschrieben in US 5,250,423.

L-Lysin produzierende Mikrorganismen besitzen typischerweise eine "feed back" resistente bzw. desensibilisierte Aspartatkinase. Unter "feed back" resistenten Aspartatkinasen versteht man Aspartatkinasen (LysC), die im Vergleich zur Wildform (Wildtyp) eine geringere Empfindlichkeit gegenüber der Hemmung durch Mischungen von Lysin und Threonin oder Mischungen von AEC (Aminoethylcystein) und Threonin oder Lysin allein oder AEC allein aufweisen. Die für diese im Vergleich zum Wildtyp desensibilisierten Aspartatkinasen kodierenden Gene bzw. Allele werden auch als lysC^{FBR}-Allele bezeichnet. Im Falle der Aspartatkinasen der Art Corynebacterium glutamicum ist der Stamm ATCC13032 der geeignete Wildtyp. Im Stand der Technik sind zahlreiche lysC^{FBR}-Allele beschrieben, die für Aspartatkinasevarianten kodieren, welche Aminosäureaustausche im Vergleich zum Wildtypprotein besitzen. Bei Bakterien der Gattung Corynebacterium wird das lysC-Gen auch als ask-Gen bezeichnet. Bei Enterobacteriaceae wird die vom lysC-Gen kodierte Aspartatkinase auch als Aspartokinase III bezeichnet.

Eine ausführliche Liste mit Angaben welche Aminosäureaustausche im Aspatatkinaseprotein von Corynebacterium glutamicum zu einer Desensibilisierung führen ist unter anderem in der WO2009141330 enthalten. Bevorzugt werden Aspartatkinasevarianten, welche folgende Aminosäureaustausche tragen, ausgewählt aus der Gruppe: an Position 380 der Aminosäuresequenz L-Isoleucin anstelle von L-Threonin und gegebenenfalls an Position 381 L-Phenylalanin anstelle L-Serin, an Position 311 L-Isoleucin anstelle L-Threonin und an Position 279 L-Threonin anstelle L-Alanin.

Ein L-Methionin ausscheidender bzw. produzierender Stamm der Art Corynebacterium glutamicum, der als Ausgangsstamm für die erfindungsgemäß die Transhydrogenase überexprimierenden Mikroorganismen dienen kann, ist beispielsweise

Corynebacterium glutamicum DSM 17322 beschrieben in WO 2007/011939.

Bekannte Vertreter L- Tryptophan produzierender bzw. ausscheidender Stämme coryneformer Bakterien, die als Ausgangsstamm für die erfindungsgemäß die Transhydrogenase überexprimierenden Mikroorganismen dienen können, sind beispielsweise:
Corynebacterium glutamicum K76 (=Ferm BP-1847) beschrieben in US 5,563,052,
Corynebacterium glutamicum BPS13 (=Ferm BP-1777) beschrieben in US 5,605,818, und
Corynebacterium glutamicum Ferm BP-3055 beschrieben in US 5,235,940.

Bekannte Vertreter L-Valin produzierender bzw. ausscheidender Stämme coryneformer Bakterien, die als Ausgangsstamm für die erfindungsgemäß die Transhydrogenase überexprimierenden Mikroorganismen dienen können, sind beispielsweise:
Brevibacterium lactofermentum FERM BP-1763 beschrieben in US 5,188,948,
Brevibacterium lactofermentum FERM BP-3007 beschrieben in US 5,521,074,
Corynebacterium glutamicum FERM BP-3006 beschrieben in US 5,521,074, und
Corynebacterium glutamicum FERM BP-1764 beschrieben in US 5,188,948.

Bekannte Vertreter L-Isoleucin produzierender bzw. ausscheidender Stämme coryneformer Bakterien, die als Ausgangsstamm für die erfindungsgemäß die Transhydrogenase überexprimierenden Mikroorganismen dienen können, sind beispielsweise:
Brevibacterium flavum FERM BP-760 beschrieben in US 4,656,135,
Brevibacterium flavum FERM BP-2215 beschrieben in US 5,294,547, und
Corynebacterium glutamicum FERM BP-758 beschrieben in US 4,656,135.

Bekannte Vertreter L-Homoserin produzierender bzw. ausscheidender Stämme coryneformer Bakterien, die als Ausgangsstamm für die erfindungsgemäß die Transhydrogenase überexprimierenden Mikroorganismen dienen können, sind beispielsweise:
Micrococcus glutamicus ATCC 14296
beschrieben in US 3,189,526 und
Micrococcus glutamicus ATCC 14297
beschrieben in US 3,189,526.

Cadaverin produzierende bzw. ausscheidende Mikroorganismen sind beispielsweise in der WO 2007/113127 beschrieben.

Bekannte Vertreter L-Threonin produzierender bzw. ausscheidender Stämme der Gattung Escherichia, insbesondere der Art Escherichia coli, die als Ausgangsstamm für die erfindungsgemäß die Transhydrogenase überexprimierenden Mikroorganismen dienen können, sind beispielsweise:
- Escherichia coli H4581 (EP 0 301 572)
- Escherichia coli KY10935 (Bioscience Biotechnology and Biochemistry 61(11):1877-1882 (1997)
- Escherichia coli VNIIgenetika MG442 (US-A-4,278,765)
- Escherichia coli VNIIgenetika M1 (US-A-4,321,325)
- Escherichia coli VNIIgenetika 472T23 (US-A-5,631,157)
- Escherichia coli BKIIM B-3996 (US-A-5,175,107)
- Escherichia coli kat 13 (WO 98/04715)
- Escherichia coli KCCM-10132 (WO 00/09660)

Bekannte Vertreter L-Threonin produzierender bzw. ausscheidender Stämme der Gattung Serratia, insbesondere der Art Serratia marcescens, die als Ausgangsstamm für die erfindungsgemäß die Transhydrogenase überexprimierenden Mikroorganismen dienen können, sind beispielsweise:
- Serratia marcescens HNr21 (Applied and Environmental Microbiology 38(6): 1045-1051 (1979))
- Serratia marcescens TLr156 (Gene 57(2-3): 151-158 (1987))
- Serratia marcescens T-2000 (Applied Biochemistry and Biotechnology 37(3): 255-265 (1992)).

Bekannte Vertreter L-Tryptophan produzierender bzw. ausscheidender Stämme der Gattung Escherichia, insbesondere der Art Escherichia coli, die als Ausgangsstamm für die erfindungsgemäß die Transhydrogenase überexprimierenden Mikroorganismen dienen können, sind beispielsweise:
- Escherichia coli JP4735/pMU3028 (US5,756,345)
- Escherichia coli JP6015/pMU91 (US5,756,345)
- Escherichia coli SV164(pGH5) (WO94/08031)
- E. coli AGX17(pGX44) (NRRL B-12263) (US4,371,614)
- E. coli AGX6(pGX50)aroP (NRRL B-12264) (US4,371,614)
- Escherichia coli AGX17/pGX50,pACKG4-pps (WO97/08333)
- Escherichia coli ATCC 31743 (CA1182409)
- E. coli C534/pD2310,pDM136 (ATCC 39795) (WO87/01130
- Escherichia coli JB102/p5LRPS2 (US5,939,295).

Bekannter Vertreter L-Homoserin produzierender bzw. ausscheidender Stämme der Gattung Escherichia, insbesondere der Art Escherichia coli, die als Ausgangsstamm für die erfindungsgemäß die Transhydrogenase überexprimierenden Mikroorganismen dienen können, ist beispielsweise:
Escherichia coli NZ10rhtA23/pAL4 (US 6, 960, 455) .

Bekannte Vertreter L-Lysin produzierender bzw. ausscheidender Stämme der Gattung Escherichia, insbesondere der Art Escherichia coli, die als Ausgangsstamm für die erfindungsgemäß die Transhydrogenase überexprimierenden Mikroorganismen dienen können, sind beispielsweise:
- Escherichia coli pDA1/TOC21R (= CNCM 1-167) (FR-A-2511032),
- Escherichia coli NRRL B-12199 (US4,346,170)
- Escherichia coli NRRL B-12185 (US4,346,170).

Eine ausführliche Liste mit Angaben welche Aminosäureaustausche im Aspartatkinase III Protein von Escherichia coli zu einer Desensibilisierung gegenüber der Hemmung durch L-Lysin führen ist in unter anderem in der EP 0 834 559 A1 auf Seite 3 (Zeilen 29 bis 41) enthalten. Bevorzugt wird eine Aspartkinasevariante, welche an Position 323 der Aminosäuresequenz L-Asparaginsäure anstelle Glycin und/oder an Position 318 L-Isoleucin anstelle L-Methionin enthält.

Bekannter Vertreter L-Valin produzierender bzw. ausscheidender Stämme der Gattung Escherichia, insbesondere der Art Escherichia coli, die als Ausgangsstamm für die erfindungsgemäß die Transhydrogenase überexprimierenden Mikroorganismen dienen können, ist beispielsweise:
- Escherichia coli AJ11502 (NRRL B-12288) (US-A-4391907).

Unter einem Polypeptid mit der Aktivität einer Transhydrogenase versteht man ein ein Enzym, das in der Lage ist, NADH in NADPH und umgekehrt umzuwandeln.

In den öffentlichen Datenbanken, wie beispielsweise der Datenbank UniProtKB (Universal Protein Resource Knowledgebase), sind Transhydrogenasen verschiedenster Lebewesen beschrieben. Die Datenbank UniProtKB wird vom UniProt Konsortium unterhalten, dem das European Bioinformatics Institute (EBI, Wellcome Trust, Hinxton Cambridge, United Kingdom), das Swiss Institute of Bioinformatics (SIB, Centre Medical Universitaire, Genf, Schweiz) und die Protein Information Resource (PIR, Georgetown University, Washington, DC, US) angehören.

Die Gene für eine Transhydrogenase können aus den Organismen mithilfe der Polymerase-Kettenreaktion (PCR) unter Verwendung geeigneter Primer isoliert werden. Anleitungen findet man unter anderem im Laborhandbuch "PCR" von Newton und Graham (Spektrum Akademischer Verlag, Heidelberg, Deutschland, 1994)und in der WO 2006/100211 auf Seite 14 bis 17.

Für die Maßnahmen der Erfindung werden bevorzugt für die Transhydrogenase aus Corynebacterien kodierende Gene eingesetzt. Besonders bevorzugt werden Gene eingesetzt, die für Polypeptide mit Aktivität einer Transhydrogenase kodieren, deren Aminosäuresequenz ≥ (mindestens) ≥ 80%, ≥ 90%, ≥ 92%, ≥ 94%, ≥ 96%, ≥ 97%, ≥ 98%, ≥ 99%, identisch sind mit der Aminosäuresequenz ausgewählt aus SEQ ID NO: 2. Während der Untersuchungen, die zur vorliegenden Erfindung führten, wurde das für die Transhydrogenase kodierende Polynukleotid von Corynebakterium glutamicum identifiziert.

Die Sequenz des Gens ist unter Seq ID No.1 hinterlegt.

Ein Gen ist chemisch gesehen ein Polynukleotid. Ein Polynukleotid, das ein Protein/Polypeptide kodiert wird hier synonym zum Begriff "Gen" gebraucht.

In einer bevorzugten Ausführungsform des erfindungsgemäßen Verfahrens, bzw. des erfindungsgemäßen Mikroorganismus überexprimiert dieser ein Gen kodierend für ein Polypeptid ausgewählt aus den folgenden i) bis vi):
i) ein Polypeptid bestehend aus oder enthaltend die in SEQ ID NO: 2 gezeigte Aminosäuresequenz;
ii) ein Polypeptid mit einer Aminosäuresequenz, die zu wenigstens 80%, wenigstens 85%, wenigstens 90%, wenigstens 95%, wenigstens 98% oder wenigstens 99% identisch ist mit der Aminosäuresequenz von i), d.h. wobei wenigstens 80%, wenigstens 85%, wenigstens 90%, wenigstens 95%, wenigstens 98% oder wenigstens 99% der Aminosäurepositionen identisch zu jenen der SEQ ID NO: 2 sind;
iii) ein Polypeptid wie unter i) oder ii) beschrieben, wobei das Polypeptid die Aktivität einer Transhydrogenase aufweist;
iv) ein Polypeptid, das eine Aminosäuresequenz enthaltend eine Deletion, Substitution, Insertion und/oder Addition von 1 bis 90, 1 bis 45, 1 bis 23, 1 bis 12, 1 bis 6 Aminosäureresten bezüglich der in SEQ ID NO: 2 gezeigten Aminosäuresequenz aufweist;
v) ein Polypeptid wie unter iv) beschrieben, wobei das Polypeptid die Aktivität einer Transhydrogenase aufweist;
vi) ein Polypeptid wie unter v) beschrieben, wobei das Polypeptid die Aktivität einer Transhydrogenase aufweist und die Deletion, Substitution, Insertion und/oder Addition von Aminosäureresten die Aktivität im Wesentlichen nicht beeinflusst;

Die prozentuale Identität wird vorzugsweise über die gesamte Länge der Aminosäure oder Nukleinsäureregion berechnet. Eine Reihe von Programmen, die auf einer Vielzahl von Algorithmen beruhen, steht dem Fachmann für den Sequenzvergleich zur Verfügung. In diesem Zusammenhang ergeben die Algorithmen von Needleman und Wunsch oder Smith und Waterman besonders verlässliche Ergebnisse. Für das Alignment der Sequenzen stehen das Programm PileUp (J. Mol. Evolution., 25, 351-360, 1987, Higgins et al., CABIOS, 5 1989: 151-153 ) oder die Programme Gap und BestFit [Needleman and Wunsch (J. Mol. Biol. 48; 443-453 (1970)) und Smith and Waterman (Adv. Appl. Math. 2; 482-489 (1981))], die zum Softwarepaket GCG gehören [Genetics Computer Group, 575 Science Drive, Madison, Wisconsin, USA 53711 (1991)] zur Verfügung. Die oben aufgeführten Prozentwerte für die Sequenzidentität werden vorzugsweise mit dem Programm GAP über die gesamte Sequenzregion berechnet.

Gegebenenfalls werden konservative Aminosäureaustausche bevorzugt. Bei den aromatischen Aminosäuren spricht man von konservativen Austauschen, wenn Phenylalanin, Tryptophan und Tyrosin gegeneinander ausgetauscht werden. Bei den hydrophoben Aminosäuren spricht man von konservativen Austauschen, wenn Leucin, Isoleucin und Valin gegeneinander ausgetauscht werden. Bei den polaren Aminosäuren spricht man von konservativen Austauschen, wenn Glutamin und Asparagin gegeneinander ausgetauscht werden. Bei den basischen Aminosäuren spricht man von konservativen Austauschen, wenn Arginin, Lysin und Histidin gegeneinander ausgetauscht werden. Bei den sauren Aminosäuren spricht man von konservativen Austauschen, wenn Asparaginsäure und Glutaminsäure gegeneinander ausgetauscht werden. Bei den Hydroxyl-Gruppen enthaltenden Aminosäuren spricht man von konservativen Austauschen, wenn Serin und Threonin gegeneinander ausgetauscht werden.

Besonders bevorzugte Ausgestaltungen des erfindungsgemäßen Verfahrens betreffen daher die fermentative Herstellung von L-Lysin oder L-Methionin unter Verwendung eines Mikroorganismus der Gattung Corynebacterium oder Escherichia, dadurch gekennzeichnet, dass man folgende Schritte durchführt
a) Fermentation eines eine organisch-chemische Verbindung produzierenden Mikroorganismus, wobei in dem Mikroorganismus ein Polynukleotid überexprimiert vorliegt, wobei das Polynukleotid für ein Polypeptid gemäß SEQ ID NO:2 einschließlich 1 bis 12, 1 bis 6 Deletionen, Substitutionen, Insertionen und/oder Additionen kodiert, und wobei die Fermentation in einem Fermentationsmedium unter Bildung einer Fermentationsbrühe abläuft;
b) Anreichern der organisch-chemischen Verbindung in der Fermentationsbrühe aus a).

Besonders bevorzugte Ausgestaltungen des erfindungsgemäßen Verfahrens betreffen weiterhin die fermentative Herstellung von L-Lysin oder L-Methionin unter Verwendung eines Mikroorganismus der Gattung Corynebacterium oder Escherichia, dadurch gekennzeichnet, dass man folgende Schritte durchführt
a) Fermentation eines eine organisch-chemische Verbindung produzierenden Mikroorganismus, wobei in dem Mikroorganismus ein Polynukleotid überexprimiert vorliegt, wobei das Polynukleotid für ein Polypeptid gemäß SEQ ID NO:2 einschließlich 1 bis 12, 1 bis 6 Deletionen, Substitutionen, Insertionen und/oder Additionen kodiert,
   wobei das Polypeptid die Aktivität einer Transhydrogenase aufweist und wobei die Fermentation in einem Fermentationsmedium unter Bildung einer Fermentationsbrühe abläuft;
b) Anreichern der organisch-chemischen Verbindung in der Fermentationsbrühe aus a).

Weiterhin können Polynukleotide verwendet werden, die mit der zu SEQ ID NO:1, bevorzugt der Kodierregion von SEQ ID NO: 1, komplementären Nukleotidsequenz unter stringenten Bedingungen hybridisieren und für ein Polypeptid, welches die Aktivität einer Transhydrogenase aufweist, kodieren.

Anleitungen zur Hybridisierung von Nukleinsäuren beziehungsweise Polynukleotiden findet der Fachmann unter anderem im Handbuch "The DIG System Users Guide for Filter Hybridization" der Firma Boehringer Mannheim GmbH (Mannheim, Deutschland, 1993) und bei Liebl et al.

(International Journal of Systematic Bacteriology 41: 255-260 (1991)). Die Hybridisierung findet unter stringenten Bedingungen statt, das heißt, es werden nur Hybride gebildet, bei denen die Sonde d.h. ein Polynukleotid umfassend die zu SEQ ID NO: 1, bevorzugt die Kodierregion von SEQ ID NO: 1, komplementäre Nukleotidsequenz und die Zielsequenz, d. h. die mit der Sonde behandelten beziehungsweise identifizierten Polynukleotide, mindestens 80% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Nukleotidsequenz der eingesetzten Sonde aufweisen. Solche Hybride sind weniger stabil und werden durch Waschen unter stringenten Bedingungen entfernt. Dies kann beispielsweise durch Senken der Salzkonzentration auf 2x SSC oder 1x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich, die Salzkonzentration bis auf eine Konzentration entsprechend 0,2x SSC oder 0,1x SSC zu senken. Gegebenenfalls enthält der SSC-Puffer Natriumdodecylsulfat (SDS) in einer Konzentration von 0,1%. Durch schrittweise Erhöhung der Hybridisierungstemperatur in Schritten von ca. 1 - 2°C von 50°C auf 68°C können Polynukleotidfragmente isoliert werden, die mindestens 80%, mindestens 90%, mindestens 92%, mindestens 94%, mindestens 96%, mindestens 97%, mindestens 98% oder mindestens 99%, gegebenenfalls 100% Identität zur Sequenz beziehungsweise komplementären Sequenz der eingesetzten Sonde besitzen und für Polypeptid, welches die Aktivität einer Transhydrogenase aufweist, kodieren. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558) .

Für die Maßnahmen der Erfindung wird ein für eine Transhydrogenase kodierendes Gen in einem die gewünschte(n) Aminosäure(n) produzierenden Mikroorganismus bzw. Ausgangs- oder Elternstamm überexprimiert.

Unter Überexpression versteht man allgemein eine Erhöhung der intrazellulären Konzentration oder Aktivität einer Ribonukleinsäure, eines Proteins (Polypeptids) oder eines Enzyms im Vergleich zum Ausgangsstamm (Elternstamm) oder Wildtypstamm, wenn dies der Ausgangsstamm ist. Unter einem Ausgangsstamm (Elternstamm) versteht man den Stamm, an dem die zur Überexpression führende Maßnahme durchgeführt wurde.

Bei der Überexpression werden die Methoden der rekombinanten Überexpression bevorzugt. Hierunter werden alle Methoden zusammengefasst bei denen ein Mikroorganismus unter Verwendung eines in-vitro bereitgestellten DNA-Moleküls hergestellt wird. Derartige DNA-Moleküle umfassen beispielsweise Promotoren, Expressionskassetten, Gene, Allele, Kodierregionen etc.. Diese werden durch Methoden der Transformation, Konjugation, Transduktion oder gleichartige Methoden in den gewünschten Mikroorganismus überführt.

Durch die Maßnahmen der Überexpression, wird die Aktivität (Gesamtaktivität in der Zelle) oder Konzentration des entsprechenden Polypeptids im Allgemeinen um mindestens 10%, 25%, 50%, 75%, 100%, 150%, 200%, 300%, 400% oder 500%, bevorzugt maximal bis 1000%, 2000%, 4000%, 10000% oder 20000% bezogen auf die Aktivität oder Konzentration des Polypeptids im Stamm vor der zur Überexpression führenden Maßnahme, erhöht.

Zur Erzielung einer Überexpression stehen im Stand der Technik eine Vielzahl von Methoden zur Verfügung. Hierzu gehören die Erhöhung der Kopienzahl und die Modifikation der Nukleotidsequenzen, die die Expression des Gens steuern bzw. kontrollieren. Die Transkription eines Gens wird unter anderem kontrolliert durch den Promotor und gegebenenfalls durch Proteine, welche die Transkription unterdrücken (Repressorproteine) oder fördern (Aktivatorproteine). Die Translation der gebildeten RNA wird unter anderem kontrolliert durch die Ribosomenbindungsstelle und das Startkodon. Polynukleotide bzw. DNA-Moleküle, welche einen Promotor und eine Ribosomenbindungsstelle und gegebenenfalls ein Startkodon umfassen, werden auch als Expressionskassette bezeichnet.

Die Erhöhung der Kopienzahl kann durch Plasmide erfolgen, die im Cytoplasma des Mikroorganismus replizieren. Hierzu sind im Stand der Technik eine Fülle von Plasmiden für die verschiedensten Gruppen von Mikroorganismen beschrieben, mit denen man die gewünschte Erhöhung der Kopienzahl des Gens einstellen kann. Geeignete Plasmide für die Gattung Escherichia sind beispielsweise im Handbuch Molecular Biology, Labfax (Ed.: T. A. Brown, Bios Scientific, Oxford, UK, 1991) beschrieben. Geeignete Plasmide für die Gattung Corynebacterium sind beispielsweise bei Tauch et al. (Journal of Biotechnology 104 (1-3), 27-40, (2003)), oder bei Stansen et al. (Applied and Environmental Microbiology 71, 5920-5928 (2005)) beschrieben.

Die Erhöhung der Kopienzahl um mindestens eine (1) Kopie kann weiterhin durch Einfügen weiterer Kopien in das Chromosom des Mikroorganismus erfolgen. Geeignete Methoden für die Gattung Corynebacterium sind beispielsweise in der Patentschrift WO 03/014330, WO 03/040373 und WO 04/069996 beschrieben. Geeignete Methoden für die Gattung Escherichia sind beispielsweise der Einbau einer Genkopie in die attsite des Phagen (Yu und Court, Gene 223, 77-81 (1998)), die chromosomale Amplifikation mit Hilfe des Phagen Mu, wie in der EP 0 332 448 beschrieben, oder die von Hamilton et al. (Journal of Bacteriology 174, 4617 - 4622 (1989)) oder Link et al. (Journal of Bacteriology 179, 6228-6237 (1997)) beschriebenen Methoden des Genaustausches mit Hilfe konditional replizierender Plasmide.

Die Erhöhung der Genexpression kann weiterhin durch Verwendung eines starken Promotors erzielt werden, der funktionell mit dem zu exprimierenden Gen verknüpft wird. Vorzugsweise wird ein Promotor verwendet, der stärker ist als der natürliche, d. h. der im Wildtyp oder Elternstamm vorhandene Promotor. Hierfür stehen im Stand der Technik eine Fülle von Methoden zur Verfügung.

Unter einer "funktionellen Verknüpfung" versteht man in diesem Zusammenhang die sequentielle Anordnung eines Promotors mit einem Gen, die zur Expression des Gens und deren Steuerung führt.

Geeignete Promotoren für die Gattung Corynebacterium finden sich unter anderem bei Morinaga et al. (Journal of Biotechnology 5, 305-312, (1987)), in den Patentschriften EP 0 629 699 A2, US 2007/0259408 A1 , WO 2006/069711, EP 1 881 076 A1 und EP 1 918 378 A1 und in zusammenfassenden Darstellungen wie dem "Handbook of Corynebacterium glutamicum" (Eds.: Lothar Eggeling und Michael Bott, CRC Press, Boca Raton, US (2005)) oder dem Buch "Corynebacteria, Genomics and Molecular Biology" (Ed.:Andreas Burkovski, Caister Academic Press, Norfolk, UK (2008)). Einsetzbare Promotoren sind auch in Fig. 1 des Übersichtsartikels von Patek et al. (Journal of Biotechnology 104(1-3), 311-323 (2003)) beschrieben. In gleicher Weise können die von Vasicova et al. (Journal of Bacteriology 181, 6188-6191 (1999)) beschrieben Varianten des dapA-Promotors, beispielsweise der Promotor A25 eingesetzt werden. Weiterhin kann der gap-Promotor von Corynebacterium glutamicum (EP 06007373, EP 2386650) oder Varianten des gap-Promotors (WO 2013000827) verwendet werden. Schließlich können die hinlänglich bekannten von Amann et al. (Gene 69(2), 301-315 (1988)) und Amann und Brosius (Gene 40(2-3), 183-190 (1985)) beschriebenen Promotoren T3, T7, SP6, M13, lac, tac und trc verwendet werden. Beispiele für Promotoren, die eine kontrollierte, das heißt induzierbare oder reprimierbare Expression erlauben, sind beispielsweise bei Tsuchiya und Morinaga (Bio/Technology 6, 428-430 (1988)) beschrieben.

Derartige Promotoren oder Expressionskassetten werden typischerweise im Abstand von 1 bis 1000, bevorzugt 1 bis 500, Nukleotiden stromaufwärts des ersten Nukleotids des Startkodons der Kodierregion des Gens eingesetzt.

Es ist ebenfalls möglich, mehrere Promotoren vor das gewünschte Gen zu positionieren bzw. funktionell mit dem zum exprimierenden Gen zu verknüpfen und auf diese Weise zu einer erhöhten Expression zu gelangen. Beispiele hierfür werden in der Patentschrift WO 2006/069711 beschrieben.

Die Struktur von Promotoren von Escherichia coli ist gut bekannt. Es ist daher möglich, die Stärke eines Promotors durch Modifikation seiner Sequenz mittels einem oder mehreren Austausch(en) und/oder einer oder mehrerer Insertion(en) und/oder einer oder mehrerer Deletion(en) von Nukleotiden zu erhöhen. Beispiele hierfür finden sich unter anderem in "Herder Lexikon der Biologie" (Spektrum Akademischer Verlag, Heidelberg, Deutschland (1994)).

Beispiele für die Veränderung von Promotoren zur Erhöhung der Expression in coryneformen Bakterien finden sich in der US 6,962,805 B2 und in einer Publikation von Vasicovä et al. (Bacteriol. 1999 Oct; 181(19):6188-91.). Die Verstärkung eins Zielgens durch Hinzufügen oder Ersatz eines homologen Promotors findet sich beispielsweise in der EP 1 697 526 B1.

Die Struktur der Ribosomenbindungsstelle Corynebacterium glutamicum ist ebenfalls gut bekannt und beispielsweise bei Amador (Microbiology 145, 915-924 (1999)) und in Hand- und Lehrbüchern der Genetik, wie beispielsweise "Gene und Klone" (Winnacker, Verlag Chemie, Weinheim, Deutschland (1990)) oder "Molecular Genetics of Bacteria" (Dale und Park, Wiley and Sons Ltd., Chichester, UK (2004)) beschrieben.

Zur Erzielung einer Überexpression ist es ebenfalls möglich, die Expression von Aktivatorproteinen zu steigern oder die Expression von Repressorproteinen zu verringern oder auszuschalten.

Die genannten Maßnahmen zur Überexpression können in geeigneter Weise miteinander kombiniert werden. So kann beispielsweise die Verwendung einer geeigneten Expressionskassette mit der Erhöhung der Kopienzahl und bevorzugt die Verwendung eines geeigneten Promotors mit der Erhöhung der Kopienzahl kombiniert werden.

Anleitungen zum Umgang mit DNA, Verdauung und Ligation von DNA, Transformation und Selektion von Transformanten findet man unter anderem in dem bekannten Handbuch von Sambrook et al. "Molecular Cloning: A Laboratory Manual, Second Edition (Cold Spring Harbor Laboratory Press, 1989).

Das Ausmaß der Expression beziehungsweise Überexpression kann durch Messung der Menge der vom Gen transkribierten mRNA, durch Bestimmung der Menge des Polypeptids und durch Bestimmung der Enzymaktivität festgestellt werden.

Für die Bestimmung der Menge an mRNA können unter anderem die Methode des "Northern Blotting's" und der quantitativen RT-PCR verwendet werden. Bei der quantitativen RT-PCR wird der Polymerase-Kettenreaktion eine reverse Transkription vorgeschaltet. Hierzu kann das LightCycler^{™} System der Firma Roche Diagnostics (Boehringer Mannheim GmbH, Roche Molecular Biochemicals, Mannheim, Deutschland) verwendet werden, wie beispielsweise bei Jungwirth et al. (FEMS Microbiology Letters 281, 190-197 (2008)) beschrieben. Die Konzentration des Proteins kann über 1- und 2-dimensionale Proteingelauftrennung und anschließende optische Identifizierung der Proteinkonzentration mit enstprechender Auswertesoftware im Gel bestimmt werden. Eine gebräuchliche Methode zur Präparation der Proteingele bei coryneformen Bakterien und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit ensprechender Software zur Konzentrationsbestimmung (Lohaus und Meyer (1998) Biospektrum 5:32-39; Lottspeich, Angewandte Chemie 321: 2630-2647 (1999)) bestimmt werden.

Das erfindungsgemäße Verfahren, bzw. die erfindungsgemäßen Mikroorganismen kann/können kontinuierlich - wie beispielsweise in der WO 05/021772 beschrieben- oder diskontinuierlich im batch - Verfahren (Satzkultivierung bzw. Satzverfahren) oder im fed batch (Zulaufverfahren) oder repeated fed batch Verfahren (repetitives Zulaufverfahren) zum Zwecke der Produktion der gewünschten organisch chemischen Verbindung betrieben/kultiviert werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium beziehungsweise Fermentationsmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium und Fermentationsmedium beziehungsweise Medium sind gegenseitig austauschbar.

Für das Durchführen der vorliegenden Erfindung ist kein spezielles Medium erforderlich.

Als Kohlenstoffquelle können Zucker und Kohlehydrate wie z.B. Glucose, Saccharose, Lactose, Fructose, Maltose, Melasse, Saccharose-haltige Lösungen aus der Zuckerrüben- oder Zuckerrohrverarbeitung, Stärke, Stärkehydrolysat und Cellulose, Öle und Fette, wie zum Beispiel Sojaöl, Sonnenblumenöl, Erdnußöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure oder Milchsäure verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muß weiterhin Salze beispielsweise in Form von Chloriden oder Sulfaten von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Thiamin, Biotin oder Pantothensäure zusätzlich zu den oben genannten Stoffen eingesetzt werden.

Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak beziehungsweise Ammoniakwasser oder saure Verbindungen wie Phosphorsäure oder Schwefelsäure in geeigneter Weise eingesetzt. Der pH wird im Allgemeinen auf einen Wert von 6,0 bis 8,5 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete, selektiv wirkende Stoffe, wie zum Beispiel Antibiotika hinzugefügt werden. Die Fermentation wird bevorzugt unter aeroben Bedingungen durchgeführt. Um diese aufrechtzuerhalten, werden Sauerstoff oder Sauerstoff-haltige Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Überdruck von 0,03 bis 0,2 MPa, gefahren. Die Temperatur der Kultur liegt normalerweise bei 20°C bis 45°C und vorzugsweise bei 25°C bis 40°C, besonders bevorzugt bei 30° bis 37°C. Bei batch-Verfahren wird die Kultivierung bevorzugt solange fortgesetzt, bis sich eine für die Maßnahme der Gewinnung der gewünschten organisch chemischen Verbindung ausreichende Menge, gebildet hat. Dieses Ziel wird normalerweise innerhalb von 10 Stunden bis 160 Stunden erreicht. Bei kontinuierlichen Verfahren sind längere Kultivierungszeiten möglich. Durch die Tätigkeit der Mikroorganismen kommt es zu einer Anreicherung (Akkumulation) der organisch chemischen Verbindung im Fermentationsmedium und/oder in den Zellen der Mikroorganismen.

Beispiele für geeignete Fermentationsmedien finden sich unter anderem in den Patentschriften 5,770,409, US 5,990,350, US 5,275,940, WO 2007/012078, US 5,827,698, WO 2009/043803, US 5,756,345 oder US 7,138,266.

Die Analyse von L-Aminosäuren zur Bestimmung der Konzentration zu einem oder mehreren Zeitpunkt(en) im Verlauf der Fermentation kann durch Trennung der L-Aminosäuren mittels Ionenaustauschchromatographie vorzugsweise Kationenaustauschchromatographie mit anschließender Nachsäulenderivatisierung unter Verwendung von Ninhydrin erfolgen, so wie bei Spackman et al. (Analytical Chemistry 30: 1190-1206 (1958)) beschrieben. Anstelle von Ninhydrin kann auch ortho-Phtadialdehyd zur Nachsäulenderivatisierung eingesetzt werden. Einen Übersichtsartikel zur Ionenaustauschchromatographie findet man bei Pickering (LC·GC (Magazine of Chromatographic Science) 7(6), 484-487 (1989)).

Es ist ebenfalls möglich eine Vorsäulenderivatisierung beispielsweise unter Verwendung von ortho-Phtadialdehyd oder Phenylisothiocyanat vorzunehmen und die entstandenen Aminosäurederivate durch Reversed-Phase-Chromatographie (RP) vorzugsweise in Form der Hochleistungsflüssigkeitschromatographie (HPLC) aufzutrennen. Eine derartige Methode ist beispielsweise bei Lindroth et al. (Analytical Chemistry 51: 1167-1174 (1979)) beschrieben.

Die Detektion erfolgt photometrisch (Absorption, Fluoreszenz).

Eine zusammenfassende Darstellung zur Aminosäureanalyse findet man unter anderem im Lehrbuch "Bioanalytik" von Lottspeich und Zorbas (Spektrum Akademischer Verlag, Heidelberg, Deutschland 1998).

Die Analyse von α-Ketosäuren wie KIC zur Bestimmung der Konzentration zu einem oder mehreren Zeitpunkt(en) im Verlauf der Fermentation kann durch Trennung der Ketosäuren und anderen Ausscheidungsprodukten mittels Ionenaustauschchromatographie, vorzugsweise Kationenaustauschchromatographie an einem sulfonierten Styrol-Divinylbenzol Polymeren in der H+ Form, z.B. mittels 0,025 N Schwefelsäure mit anschließender UV-Detektion bei 215nm (alternativ auch bei 230 oder 275 nm) erfolgen. Vorzugsweise kann eine REZEX RFQ - Fast Fruit H+ Säule (Phenomenex) eingesetzt werden, andere Lieferanten für die Trennphase (z.B. Aminex von BioRad) sind möglich. Analoge Trennungen sind in entsprechenden Applikationsbeispielen der Lieferanten beschrieben.

Die Leistung der erfindungsgemäßen Verfahren bzw. Fermentationsprozesse bezüglich eines oder mehrerer der Parameter ausgewählt aus der Gruppe der Konzentration (gebildete Verbindung pro Volumen), der Ausbeute (gebildete Verbindung pro verbrauchter Kohlenstoff-Quelle), der Bildung (gebildete Verbindung pro Volumen und Zeit) und der spezifischen Bildung (gebildete Verbindung pro Zelltrockenmasse bzw. Biotrockenmasse und Zeit oder gebildete Verbindung pro Zellprotein und Zeit) oder auch anderer Prozess-Parameter und Kombinationen davon, wird um mindestens 0,5%, mindestens 1%, mindestens 1,5% oder mindestens 2% erhöht, bezogen auf Verfahren bzw. Fermentationsprozesse mit Mikroorganismen, in denen die Aktivität einer Transhydrogenase erhöht vorliegt.

Durch die Maßnahmen der Fermentation erhält man eine Fermentationsbrühe, welche die gewünschte organisch chemische Verbindung, bevorzugt L-Aminosäure, enthält. Anschließend erfolgt die Bereitstellung bzw. Herstellung oder Gewinnung eines die organisch chemische Verbindung enthaltenden Produktes in flüssiger oder fester Form.

Unter einer Fermentationsbrühe versteht man ein Fermentationsmedium bzw. Nährmedium, in dem ein Mikroorganismus für eine gewisse Zeit und bei einer gewissen Temperatur kultiviert wurde. Das Fermentationsmedium beziehungsweise die während der Fermentation eingesetzten Medien enthält/enthalten sämtliche Substanzen beziehungsweise Komponenten, die eine Produktion der gewünschten Verbindung und typischerweise die Vermehrung bzw. Lebensfähigkeit sicherstellen.

Bei Abschluss der Fermentation enthält die entstandene Fermentationsbrühe dementsprechend
a) die infolge der Vermehrung der Zellen des Mikroorganismus entstandene Biomasse (Zellmasse) des Mikroorganismus,
b) die im Laufe der Fermentation gebildete gewünschte organisch chemische Verbindung,
c) die im Laufe der Fermentation gebildeten organischen Nebenprodukte, und
d) die durch die Fermentation nicht verbrauchten Bestandteile des eingesetzten Fermentationsmediums beziehungsweise der Einsatzstoffe wie beispielsweise Vitamine wie Biotin oder Salze wie Magnesiumsulfat.

Zu den organischen Nebenprodukten gehören Stoffe, die von den bei der Fermentation eingesetzten Mikroorganismen neben der jeweiligen gewünschten Verbindung erzeugt und gegebenenfalls ausgeschieden werden. Hierzu gehören auch Zucker wie zum Beispiel Trehalose.

Die Fermentationsbrühe wird dem Kulturgefäß beziehungsweise dem Fermentationsbehälter entnommen, gegebenenfalls gesammelt, und dazu verwendet, ein die organisch chemische Verbindung enthaltendes Produkt, bevorzugt ein L-Aminosäure-haltiges Produkt in flüssiger oder fester Form bereitzustellen. Hierfür wird auch der Ausdruck "Gewinnen des L-Aminosäure haltigen Produktes" verwendet. Im einfachsten Fall stellt die dem Fermentationsbehälter entnommene L-Aminosäure-haltige Fermentationsbrühe selbst das gewonnene Produkt dar.

Durch eine oder mehrere der Maßnahmen ausgewählt aus der Gruppe
teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%) Entfernung des Wassers,
teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%) Entfernung der Biomasse, wobei diese gegebenenfalls vor der Entfernung inaktiviert wird,
teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%, ≥ 99,3%, ≥ 99,7%) Entfernung der im Laufe der Fermentation gebildeten organischen Nebenprodukte, und
teilweise (> 0%) bis vollständige (100%) oder nahezu vollständige (≥ 80%, ≥ 90%, ≥ 95%, ≥ 96%, ≥ 97%, ≥ 98%, ≥ 99%, ≥ 99,3%, ≥ 99,7%) Entfernung der durch die Fermentation nicht verbrauchten Bestandteile des eingesetzten Fermentationsmediums beziehungsweise der Einsatzstoffe,
aus der Fermentationsbrühe erzielt man eine Konzentrierung bzw. Reinigung der gewünschten organisch chemischen Verbindung. Auf diese Weise werden Produkte isoliert, die einen gewünschten Gehalt an der Verbindung aufweisen.

Die teilweise (> 0% bis < 80%) bis vollständige (100%) oder nahezu vollständige (≥ 80% bis < 100%) Entfernung des Wassers (Maßnahme a)) wird auch als Trocknung bezeichnet.

In einer Variante des Verfahrens gelant man durch vollständige oder nahezu vollständige Entfernung des Wassers, der Biomasse, der organischen Nebenprodukte und der nicht verbrauchten Bestandteile des eingesetzten Fermentationsmediums gelangt man zu reinen ((≥ 80 Gew.-%, ≥ 90 Gew.-%) oder hochreinen (≥ 95 Gew.-%, ≥ 97 Gew.-%, ≥ 99% Gew.-%) Produktformen der gewünschten organisch chemischen Verbindung, bevorzugt L-Aminosäuren. Für die Maßnahmen gemäß a), b), c) oder d) sind im Stand der Technik eine Fülle von technischen Anleitungen verfügbar.

Bei Verfahren zur Herstellung von organischen Säuren oder L-Valin, L-Leucin und L-Isoleucin unter Verwendung von Bakterien der Gattung Corynebacterium werden solche Verfahren bevorzugt, bei denen Produkte erhalten werden, die keine Bestandteile der Fermentationsbrühe enthalten. Diese werden insbesondere in der Humanmedizin, in der pharmazeutischen Industrie und in der Lebensmittelindustrie verwendet.

Im Falle der Aminosäure L-Lysin sind im Stand der Technik im Wesentlichen vier verschiedene Produktformen bekannt.

Eine Gruppe L-Lysin-haltiger Produkte umfasst konzentrierte, wässrige, alkalische Lösungen von aufgereinigtem L-Lysin (EP-B-0534865). Eine weitere Gruppe, wie beispielsweise in der US 6,340,486 und US 6,465,025 beschrieben, umfasst wässrige, saure, Biomasse-haltige Konzentrate von L-Lysin-haltigen Fermentationsbrühen. Die bekannteste Gruppe fester Produkte umfasst pulverförmige oder kristalline Formen von aufgereinigtem beziehungsweise reinem L-Lysin, das typischerweise in Form eines Salzes wie zum Beispiel L-Lysin-Monohydrochlorid vorliegt. Eine weitere Gruppe fester Produktformen ist beispielsweise in der EP-B-0533039 beschrieben. Die dort beschriebene Produktform enthält neben L-Lysin den größten Teil der während der fermentativen Herstellung verwendeten und nicht verbrauchten Einsatzstoffe und gegebenfalls die Biomasse des eingesetzten Mikroorganismus mit einem Anteil von >0% - 100%.

Entsprechend der verschiedenen Produktformen kennt man verschiedenste Verfahren, bei denen aus der Fermentationsbrühe das L-Lysin-haltige Produkt oder das gereinigte L-Lysin hergestellt wird.

Zur Herstellung von festem, reinem L-Lysin werden im Wesentlichen Methoden der Ionenaustauschchromatographie gegebenfalls unter Verwendung von Aktivkohle und Methoden der Kristallisierung angewendet. Auf diese Weise erhält man die entsprechende Base oder ein entsprechendes Salz wie beispielsweise das Monohydrochlorid (Lys-HCl) oder das Lysinsulfat (Lys₂-H₂SO₄) .

In der EP-B-0534865 ist ein Verfahren zur Herstellung wässriger, basischer L-Lysin-haltiger Lösungen aus Fermentationsbrühen beschrieben. Bei dem dort beschriebenen Verfahren wird die Biomasse aus der Fermentationsbrühe abgetrennt und verworfen. Mittels einer Base wie beispielsweise Natrium-, Kalium- oder Ammoniumhydroxid wird ein pH-Wert zwischen 9 bis 11 eingestellt. Die mineralischen Bestandteile (anorganischen Salze) werden nach Konzentrierung und Abkühlung durch Kristallisation aus der Brühe abgetrennt und entweder als Dünger verwendet oder verworfen.

Bei Verfahren zur Herstellung von Lysin unter Verwendung von Bakterien der Gattung Corynebacterium werden solche Verfahren bevorzugt, bei denen Produkte erhalten werden, die Bestandteile der Fermentationsbrühe enthalten. Diese werden insbesondere als Tierfuttermitteladditive verwendet.

Je nach Anforderung kann die Biomasse ganz oder teilweise durch Separationsmethoden wie z.B. der Zentrifugation, der Filtration, dem Dekantieren oder einer Kombination hieraus aus der Fermentationsbrühe entfernt oder vollständig in ihr belassen werden. Gegebenenfalls wird die Biomasse beziehungsweise die Biomasse enthaltene Fermentationsbrühe während eines geeigneten Verfahrensschrittes inaktiviert beispielsweise durch thermische Behandlung (Erhitzen) oder durch Säurezugabe.

In einer Verfahrensweise wird die Biomasse vollständig oder nahezu vollständig entfernt, sodass keine (0 %) oder höchstens 30%, höchstens 20%, höchstens 10%, höchstens 5%, höchstens 1% oder höchstens 0,1% Biomasse im hergestellten Produkt verbleibt. In einer weiteren Verfahrensweise wird die Biomasse nicht oder nur in geringfügigen Anteilen entfernt, sodass sämtliche (100%) oder mehr als 70%, 80%, 90%, 95%, 99% oder 99,9% Biomasse im hergestellten Produkt verbleibt. In einem erfindungsgemäßen Verfahren wird dementsprechend die Biomasse in Anteilen ≥ 0% bis ≤ 100% entfernt.

Schließlich kann die nach der Fermentation erhaltene Fermentationsbrühe vor oder nach der vollständigen oder teilweisen Entfernung der Biomasse mit einer anorganischen Säure wie beispielsweise Salzsäure, Schwefelsäure oder Phosphorsäure oder organischen Säure wie beispielsweise Propionsäure auf einen sauren pH Wert eingestellt werden (GB 1,439,728 oder EP 1 331 220). Es ist gleichfalls möglich, die Fermentationsbrühe mit der vollständig enthaltenen Biomasse anzusäuern. Schließlich kann die Brühe auch durch Zusatz von Natriumbisulfit (NaHSO₃, GB 1,439,728) oder einem anderen Salz beispielsweise Ammonium-, Alkali- oder Erdalkalisalz der schwefligen Säure stabilisiert werden.

Bei der Abtrennung der Biomasse werden gegebenenfalls in der Fermentationsbrühe enthaltene organische oder anorganische Feststoffe teilweise oder ganz entfernt. Die in der Fermentationsbrühe gelösten organischen Nebenprodukte und die gelösten nicht verbrauchten Bestandteile des Fermentationsmediums (Einsatzstoffe) bleiben mindestens teilweise (> 0%), bevorzugt zu mindestens 25%, besonders bevorzugt zu mindestens 50% und ganz besonders bevorzugt zu mindestens 75% im Produkt. Gegebenenfalls bleiben diese auch vollständig (100%) oder nahezu vollständig, das heißt > 95% oder > 98% oder größer 99%, im Produkt. Enthält ein Produkt in diesem Sinn mindestens einen Teil der Bestandteile der Fermentationsbrühe, so wird dies auch mit dem Begriff "Produkt auf Fermentationsbrühebasis" umschrieben.

Anschließend wird der Brühe mit bekannten Methoden wie z.B. mit Hilfe eines Rotationsverdampfers, Dünnschichtverdampfers, Fallfilmverdampfers, durch Umkehrosmose oder durch Nanofiltration Wasser entzogen beziehungsweise eingedickt oder konzentriert. Diese aufkonzentrierte Fermentationsbrühe kann anschließend durch Methoden der Gefriertrocknung, der Sprühtrocknung, der Sprühgranulation oder durch anderweitige Verfahren wie zum Beispiel in der zirkulierenden Wirbelschicht gemäß PCT/EP2004/006655 beschrieben, zu rieselfähigen Produkten insbesondere zu einem feinteiligen Pulver oder vorzugsweise grobkörnigem Granulat aufgearbeitet werden. Gegebenenfalls wird aus dem erhaltenen Granulat durch Sieben oder Staubabtrennung ein gewünschtes Produkt isoliert.

Es ist ebenfalls möglich, die Fermentationsbrühe direkt, d. h. ohne vorherige Aufkonzentrierung durch Sprühtrocknung oder Sprühgranulation, zu trocknen.

Unter "rieselfähig" versteht man Pulver, die aus einer Serie von Glasauslaufgefäßen mit verschieden großen Auslauföffnungen mindestens aus dem Gefäß mit der Öffnung 5 mm (Millimeter) ungehindert auslaufen (Klein: Seifen, Öle, Fette, Wachse 94, 12 (1968)).

Mit "feinteilig" ist ein Pulver mit überwiegendem Anteil (> 50 %) einer Korngröße von 20 bis 200 µm Durchmesser gemeint.

Mit "grobkörnig" ist ein Produkt mit einem überwiegendem Anteil (> 50 %) einer Korngröße von 200 bis 2000 µm Durchmesser gemeint.

Die Korngrößenbestimmung kann mit Methoden der Laserbeugungsspektrometrie durchgeführt werden. Die entsprechenden Methoden sind im Lehrbuch zur "Teilchengrößenmessung in der Laborpraxis" von R. H. Müller und R. Schuhmann, Wissenschaftliche Verlagsgesellschaft Stuttgart (1996) oder im Lehrbuch "Introduction to Particle Technology" von M. Rhodes, Verlag Wiley & Sons (1998) beschrieben.

Das rieselfähige, feinteilige Pulver kann wiederum durch geeignete Kompaktier- oder Granulier-Verfahren in ein grobkörniges, gut rieselfähiges, lagerfähiges und weitgehend staubfreies Produkt überführt werden.

Der Begriff "staubfrei" bedeutet, daß das Produkt lediglich geringe Anteile (< 5 %) an Korngrößen unter 100 µm Durchmesser enthält.

"Lagerfähig", im Sinne dieser Erfindung, bedeutet ein Produkt, das mindestens ein (1) Jahr oder länger, bevorzugt mindestens 1,5 Jahre oder länger, besonders bevorzugt zwei (2) Jahre oder länger in trockener und kühler Umgebung gelagert werden kann, ohne dass ein wesentlicher Verlust (maximal 5%) der jeweiligen Aminosäure auftritt.

Ein weiterer Gegenstand der Erfindung ist ein Verfahren, das in seinen Grundzügen in der WO 2007/042363 A1 beschrieben ist. Hierfür wird unter Verwendung der erfindungsgemäß erhaltenen Fermentationsbrühe, aus der die Biomasse gegebenenfalls ganz oder teilweise abgetrennt wurde, ein Verfahren durchgeführt, das folgende Schritte umfasst:
a) den pH-Wert durch Zugabe von Schwefelsäure auf 4,0 bis 5,2, insbesondere 4,9 bis 5,1, absenkt, und ein molares Sulfat/L-Lysin-Verhältnis von 0,85 bis 1,2, bevorzugt 0,9 bis 1,0, besonders bevorzugt >0,9 bis <0,95, in der Brühe einstellt, gegebenenfalls durch Zugabe von einer weiteren oder mehreren Sulfat-haltigen Verbindung(en) und
b) das so erhaltene Gemisch durch Wasserentzug aufkonzentriert und gegebenenfalls granuliert,
   wobei gegebenenfalls vor Schritt a) eine oder beide der folgenden Maßnahmen durchgeführt wird/werden:
c) Messung des molaren Verhältnisses von Sulfat/L-Lysin zur Ermittlung der benötigten Menge an Sulfat-haltigen Verbindung(n)
d) Zusatz einer Sulfat-haltigen Verbindung ausgewählt aus der Gruppe Ammoniumsulfat, Ammoniumhydrogensulfat und Schwefelsäure in entsprechenden Verhältnissen.

Gegebenenfalls wird weiterhin vor Schritt b) ein Salz der schwefligen Säure, bevorzugt Alkalihydrogensulfit, besonders bevorzugt Natriumhydrogensulfit in einer Konzentration von 0,01 bis 0,5 Gew.-%, bevorzugt 0,1 bis 0,3 Gew.-%, besonders bevorzugt 0,1 bis 0,2 Gew.-% bezogen auf die Fermentationsbrühe hinzugesetzt.

Als bevorzugte Sulfat-haltige Verbindungen im Sinne der oben genannten Verfahrensschritte sind insbesondere Ammoniumsulfat und/oder Ammoniumhydrogensulfat oder entsprechende Mischungen von Ammoniak und Schwefelsäure und Schwefelsäure selbst zu nennen.

Das molare Sulfat/L-Lysin-Verhältnis V wird nach der Formel: V = 2 x [SO₄²⁻] / [L-Lysin] berechnet. Diese Formel berücksichtigt die Tatsache, dass das SO₄²- Anion, bzw. die Schwefelsäure zweiwertig ist. Ein Verhältnis V = 1 bedeutet, dass ein stöchiometrisch zusammengesetztes Lys₂-H₂SO₄) vorliegt, während bei einem Verhältnis von V = 0,9 ein 10%iger Sulfatmangel und bei einem Verhältnis von V = 1,1 ein 10% Sulfatüberschuss gefunden wird.

Vorteilhaft bei der Granulation oder Kompaktierung ist der Einsatz von üblichen organischen oder anorganischen Hilfsstoffen, beziehungsweise Trägern wie Stärke, Gelatine, Cellulosederivaten oder ähnlichen Stoffen, wie sie üblicherweise in der Lebensmittel- oder Futterverarbeitung als Binde-, Gelier-, oder Verdickungsmittel Verwendung finden, oder von weiteren Stoffen wie zum Beispiel Kieselsäuren, Silikaten (EP0743016A) und Stearaten.

Weiterhin ist es vorteilhaft, die Oberfläche der erhaltenen Granulate mit Ölen oder Fetten zu behandeln so wie es in der WO 04/054381 beschrieben ist. Als Öle können Mineralöle, pflanzliche Öle oder Mischungen pflanzlicher Öle verwendet werden. Beispiele für derartige Öle sind Sojaöl, Olivenöl, Sojaöl/Lecithingemische. In gleicher Weise sind auch Silikonöle, Polyethylenglykole oder Hydroxyethylcellulose geeignet. Durch die Behandlung der Oberflächen mit den genannten Ölen erzielt man eine erhöhte Abriebfestigkeit des Produktes und einen Verringerung des Staubanteils. Der Gehalt an Öl im Produkt beträgt 0,02 bis 2,0 Gew.-%, bevorzugt 0,02 bis 1,0 Gew.-%, und ganz besonders bevorzugt 0,2 bis 1,0 Gew.-% bezogen auf die Gesamtmenge des Futtermitteladditivs.

Bevorzugt sind Produkte mit einem Anteil von ≥ 97 Gew.-% einer Korngröße von 100 bis 1800 µm oder einem Anteil von ≥ 95 Gew.-% einer Korngröße von 300 bis 1800 µm Durchmesser. Der Anteil an Staub, d. h. Partikeln mit einer Korngrösse < 100 µm, liegt bevorzugt bei > 0 bis 1 Gew.- besonders bevorzugt bei maximal 0,5 Gew.-%.

Alternativ kann das Produkt aber auch auf einen in der Futtermittelverarbeitung bekannten und üblichen organischen oder anorganischen Trägerstoff wie zum Beispiel Kieselsäuren, Silikate, Schrote, Kleien, Mehle, Stärken, Zucker oder andere aufgezogen und/oder mit üblichen Verdickungs- oder Bindemitteln vermischt und stabilisiert werden. Anwendungsbeispiele und Verfahren hierzu sind in der Literatur (Die Mühle + Mischfuttertechnik 132 (1995) 49, Seite 817) beschrieben.

Schließlich kann das Produkt auch durch Beschichtungsverfahren ("Coating") mit Filmbildnern wie beispielsweise Metallcarbonaten, Kieselsäuren, Silikaten, Alginaten, Stearaten, Stärken, Gummis und Celluloseether, wie in der DE-C-4100920 beschrieben, in einen Zustand gebracht werden, in dem es stabil gegenüber der Verdauung durch Tiermägen, insbesondere dem Magen von Wiederkäuern, ist.

Zur Einstellung einer gewünschten L-Lysin Konzentration im Produkt kann je nach Anforderung das L-Lysin während des Verfahrens in Form eines Konzentrates oder gegebenenfalls einer weitgehend reinen Substanz beziehungsweise dessen Salz in flüssiger oder fester Form hinzugefügt werden. Diese können einzeln oder als Mischungen zur erhaltenen oder aufkonzentrierten Fermentationsbrühe, oder auch während des Trocknungs- oder Granulationsprozesses, hinzugefügt werden.

Ein weiterer Gegenstand der Erfindung ist ein Verfahren zur Herstellung eines festen Lysin-haltigen Produktes, das in seinen Grundzügen in der US 20050220933 beschrieben ist. Hierbei wird unter Verwendung der erfindungsgemäß erhaltenen Fermentationsbrühe ein Verfahren durchgeführt, das folgende Schritte umfasst:
a) Filtration der Fermentationsbrühe, bevorzugt mit einem Membranfilter, so dass ein Biomasse-haltiger Schlamm und ein Filtrat erhalten wird,
b) Aufkonzentration des Filtrates, bevorzugt so, dass ein Feststoffgehalt von 48 bis 52 Gew.-% erhalten wird,
c) Granulation des in Schritt b) erhaltenen Konzentrates, bevorzugt bei einer Temperatur von 50°C bis 62°C, und
d) Beschichtung des in c) erhaltenen Granulates mit einem oder mehreren der Beschichtungsmittel (coating agent(s))

Die Aufkonzentrierung des Filtrates in Schritt b) kann auch derart erfolgen, dass ein Feststoffgehalt von 52 bis 55 Gew.-%, von 55 bis 58 Gew.-% oder 58 bis 61 Gew.-% erhalten wird.

Zur Beschichtung in Schritt d) werden bevorzugt Beschichtungsmittel verwendet, welche ausgewählt werden aus der Gruppe, bestehend aus
d1) der in Schritt a) erhaltenen Biomasse,
d2) einer L-Lysin-haltigen Verbindung, bevorzugt ausgewählt aus der Gruppe L-Lysinhydrochlorid oder L-Lysinsulfat,
d3) einem im wesentlichen L-Lysin-freien Stoff mit L-Lysingehalt < 1 Gew.-%, bevorzugt < 0,5 Gew.-%, bevorzugt ausgewählt aus der Gruppe Stärke, Karageenan, Agar, Kieselsäuren, Silikate, Schrote, Kleien und Mehle, und
d4) einem wasserabstoßenden Stoff, bevorzugt ausgewählt aus der Gruppe Öle, Polyethylenglykole und flüssige Paraffine.

Durch die Maßnahmen entsprechend den Schritten d1) bis d4), insbesondere d1) bis d3), wird der Gehalt an L-Lysin auf einen gewünschten Wert eingestellt.

Bei der Herstellung L-Lysin-haltiger Produkte wird das Verhältnis der Ionen bevorzugt so eingestellt, dass das molare Ionenverhältnis entsprechend nachstehender Formel 2x [SO₄²⁻] + [Cl⁻] - [NH₄⁺] - [Na⁺] - [K⁺] -2x [Mg²⁺] -2x [Ca²⁺] / [L-Lys]

0,68 bis 0,95, bevorzugt 0,68 bis 0,90, besonders bevorzugt 0,68 bis 0,86 ergibt, so wie von Kushiki et al. in der US 20030152633 beschrieben.

Im Falle des L-Lysins hat das auf diese Weise hergestellte feste Produkt auf Fermentationsbrühebasis einen Lysingehalt (als Lysinbase) von 10 Gew.-% bis 70 Gew.-% oder 20 Gew.-% bis 70 Gew.-%, bevorzugt 30 Gew.-% bis 70 Gew.-% und ganz besonders bevorzugt von 40 Gew.-% bis 70 Gew.-% bezogen auf die Trockenmasse des Produkts. Maximale Gehalte an Lysinbase von 71 Gew.-%, 72 Gew.-%, 73 Gew.-% sind ebenfalls möglich.

Der Wassergehalt des L-Lysin-haltigen, festen Produktes beträgt bis zu 5 Gew.-%, bevorzugt bis zu 4 Gew.-%, und besonders bevorzugt weniger als 3 Gew.-%.

Der Stamm DM1547 wurde am 16. Januar 2001 bei der Deutschen Sammlung für Mikroorganismen und Zellkulturen unter der Zugangsnummer DSM13994 hinterlegt.

### Beschreibung der Figuren

**Figur 1** zeigtPlasmid pK18msb_Pg3_lpdA.
**Figur 2** zeigt Plasmid p28-1::lpdA.

### Beispiele

### Beispiel 1:

Das Genprodukt des lpdA-Gens (cg0790) aus Corynebacterium glutamicum wurde wie im Fall des LpdA-Proteins aus Mycobacterium tuberculosis (Argyrou et al., 2004) bei einer überwiegend automatischen Genomannotation als mögliche Dihydroliponamid-Dehydrogenase zugehörig der Flavoprotein-Disulfid-Reduktase-(FDR)-Familie, deren Mitglieder trotz ihrer Vielfalt an katalysierten Redoxreaktionen eine allgemein hohe Homologie auf Sequenz- und Strukturebene zueinander aufweisen, klassifiziert. Durch die bioinformatische Analyse von C. glutamicum LpdA mit Hilfe der blastp-Funktion des BLAST-Programmes (Basic Local Alignment Search Tool) wurde die Homologie dieses Proteins zu dem bereits charakterisierten LpdA aus Mycobacterium tuberculosis und anderen Flavoprotein-Disulfid-Reduktasen aus C. glutamicum und verschiedenen weiteren Organismen ermittelt.

### Beispiel 2:

### Konstruktion des Austauschvektors pK18msb_Pg3_lpdA

Die Nukleotidsequenz des Genoms von Corynebacterium glutamicum ATCC13032 ist bei Ikeda und Nakagawa (Applied Microbiology and Biotechnology 62, 99-109 (2003)), in der EP 1 108 790 und bei Kalinowski et al. (Journal of Biotechnolgy 104(1-3), (2003)) beschrieben.

Die Nukleotidsequenz des Genoms von Corynebacterium glutamicum ist ebenfalls in der Datenbank des National Center for Biotechnology Information (NCBI) der National Library of Medicine (Bethesda, MD, USA), in der DNA Data Bank of Japan (DDBJ, Mishima, Japan) oder in der Nukleotidsequenz-Datenbank der European Molecular Biologies Laboratories (EMBL, Heidelberg, Deutschland bzw. Cambridge, UK) verfügbar.

Ausgehend von der Genomsequenz von Corynebacterium glutamicum ATCC13032 wurde ein ca.1,7 kb großes DNA-Fragment bei der Firma GeneArt (Regensburg, Deutschland) synthetisiert (SEQ ID NO: 3), welches neben dem Pgap3-Promotor (DE102011118019.6) die flankierenden Sequenzen für eine Insertion des Promotors vor das native lpdA-Gen trägt. Das Fragment wurde über die terminal eingeführten Schnittstellen XmaI und XbaI durch XmaI und XbaI -Spaltung geschnitten und anschließend in den ebenfalls XmaI / XbaI - geschnittenen von Schäfer et al. (Gene, 145, 69-73 (1994)) beschriebenen, mobilisierbaren Vektor pK18mobsacB kloniert. Das Plasmid trägt die Bezeichnung pK18msb_Pg3_lpdA und ist in Figur 1 dargestellt.

### Beispiel 3:

### Konstruktion des Vektors pZ8-l_lpdA

Das lpdA Gen aus C. glutamicum (cg0790) wurde mit Hilfe zweier Primer amplifiziert, durch die das Gen upstream mit einer EcoRI- und downstream mit einer SalI-Schnittstelle versehen wurde. Aufgrund der für C.glutamicum bekannten Sequenz des lpdA-Gens wurden folgende Oligonukleotide als Primer verwendet:
lpdA_pZ8-1-1 (SEQ ID NO:4) :
   5' GGTGGTGAATTCAAAGGAGGACAACCATGGCAAAGAGGATCGTAAT 3'
lpdA_pZ8-1-2 (SEQ ID NO:5) :
   5' GGTGGTGTCGACTTAGCCTAGATCATCATGTT 3'

Die dargestellten Primer wurden von der Firma MWG Biotech (Ebersberg, Deutschland) synthetisiert.

Sie ermöglichen die Amplifizierung eines 1448 bp langen DNA-Abschnittes gemäß SEQ ID NO:6.

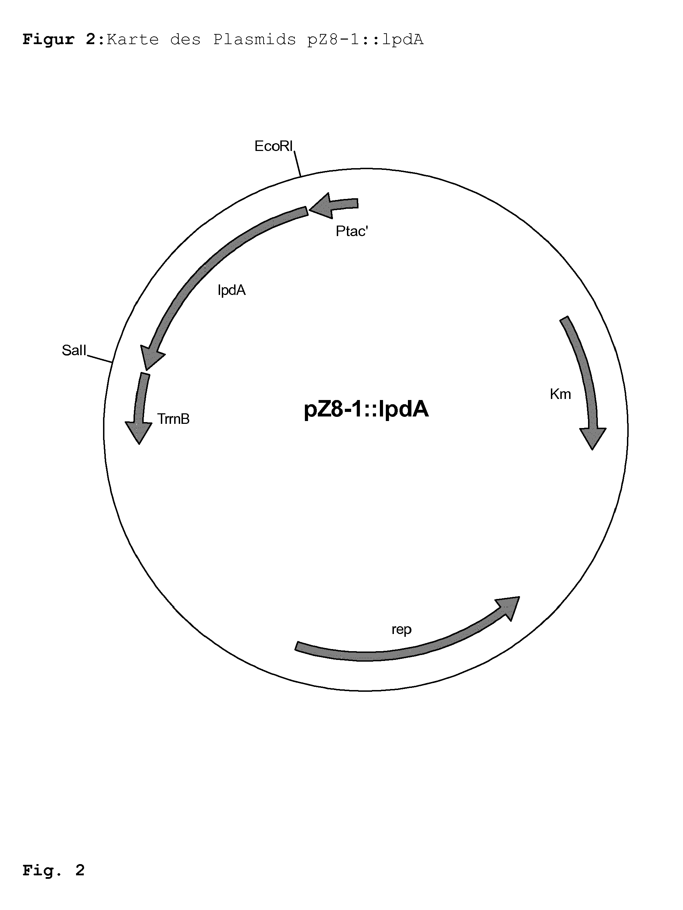

Das PCR-Produkt wurde mit dem Nucleospin® Extract II Kit aufgereinigt. Das Fragment wurde über die terminal eingeführten Schnittstellen EcoRI und SalI durch EcoRI und SalI -Spaltung geschnitten und anschließend in den ebenfalls EcoRI / SalI -geschnittenen Vektor pZ8-1 (DE3841454A1; E.coli DH5/pZ8-1 wurde bei der Deutschen Stammsammlung für Mikroorganismen unter der Nummer DSM 4939 hinterlegt) mit Hilfe von T4 DNA-Ligase kloniert. Das Plasmid trägt die Bezeichnung pZ8-1::lpdA und ist in Figur 2 dargestellt.

### Beispiel 4:

### Herstellung des Stammes DM1547_Pg3_lpdA

Die Mutation Pg3_lpdA soll in den Stamm Corynebacterium glutamicum DM1547 eingebracht werden. Der Stamm DM1547 ist eine Aminoethylcystein-resistente Mutante von Corynebacterium glutamicum ATCC13032. Sie ist unter der Bezeichnung DSM13994 am 16.01.2001 bei der Deutschen Sammlung für Mikroorganismen und Zellkulturen (DSMZ, Braunschweig, Deutschland) hinterlegt.

Der in Beispiel 2 beschriebene Vektor pK18msb_Pg3_lpdA wurden mit der Elektroporationsmethode von Liebl et al. (FEMS Microbiological Letters, 53:299-303 (1989)) in Corynebacterium glutamicum DM1547 elektroporiert. Die Selektion von Plasmid-tragenden Zellen erfolgte durch Ausplattieren des Elektroporationsansatzes auf LB Agar (Sambrook et al., Molecular cloning: a laboratory manual. 2nd Ed. Cold Spring Harbor Laboratory Press, Cold Spring Harbor, N.Y., 1989), der mit 25 mg/l Kanamycin supplementiert worden war. Der Vektor kann in DM1547 nicht selbständig replizieren und bleibt nur dann in der Zelle erhalten, wenn er als Folge eines Rekombinationsereignisses im Chromosom integriert vorliegt. Die Selektion von Transkonjuganten, d.h. von Klonen mit integriertem pK18msb_Pg3_lpdA erfolgte durch Ausplattieren des Konjugationsansatzes auf LB-Agar, der mit 25 mg/l Kanamycin und 50 mg/l Nalidixinsäure supplementiert worden war. Kanamycin-resistente Transkonjuganten wurden anschließend auf mit Kanamycin (25 mg/l) supplementierten LB-Agarplatten ausgestrichen und für 24 Stunden bei 33°C inkubiert. Zur Selektion von Mutanten, bei denen als Folge eines zweiten Rekombinationsereignisses die Exzision des Plasmides stattgefunden hatte, wurden die Klone 30 Stunden unselektiv in LB-Flüssigmedium kultiviert, anschließend auf LB-Agar, der mit 10% Sucrose supplementiert worden war ausgestrichen und 24 Stunden bei 33°C bebrütet.

Das Plasmid pK18msb_Pg3_lpdA enthält ebenso wie das Ausgangsplasmid pK18mobsacB neben dem Kanamycin-Resistenzgen eine Kopie des für die Levan-Sucrase aus Bacillus subtilis kodierenden sacB-Gens. Die durch Sucrose induzierbare Expression des sacB-Gens führt zur Bildung der Levan-Sucrase, welche die Synthese des für C. glutamicum toxischen Produktes Levan katalysiert.

Auf mit Sucrose supplementiertem LB-Agar wachsen daher nur solche Klone, bei denen das integrierte pK18msb_Pg3_1pdA als Folge eines zweiten Rekombinationsereignisses exzisiert hat. In Abhängigkeit von der Lage des zweiten Rekombinationsereignisses in bezug auf den Mutationsort findet bei der Exzision der Allelaustausch bzw. der Einbau der Mutation statt oder es verbleibt die ursprüngliche Kopie im Chromosom des Wirtes.

Anschließend wurde jeweils ein Klon gesucht, bei dem der gewünschte Austausch, d. h. der Einbau der Mutation Pg3_1pdA erfolgt war.

Hierzu wurden jeweils 20 Klone mit dem Phänotyp "Wachstum in Gegenwart von Sucrose" und "Nicht-Wachstum in Gegenwart von Kanamycin auf Integration der Mutation Pg3_lpdA unter Anwendung der Polymerase-Kettenreaktion (PCR) überprüft.

Hierfür wurden folgende synthetische Oligonukleotide (Primer) verwendet:
lpdA_1.p (SEQ ID NO: 8):
   5' AACACGTCCCAGGATCAATG 3'
lpdA_2.p (SEQ ID NO: 9):
   5' TTTCGCAAGGTCCTTCACAC 3'

Die dargestellten Primer wurden von der Firma MWG Biotech (Ebersberg, Deutschland) synthetisiert. Die PCR-Reaktionen wurden mit dem Taq PCR Core Kit von Quiagen (Hilden, Deutschland), enthaltend die Taq DNA Polymerase aus *Thermus aquaticus,* in einem Mastercycler der Firma Eppendorf (Hamburg, Deutschland)durchgeführt. Die Bedingungen im Reaktionsansatz wurden nach Angaben des Herstellers eingestellt. Der PCR-Ansatz wurde zuerst einer einleitenden Denaturierung bei 94°C für 2 Minuten unterzogen. Darauf folgten sich 35x wiederholend ein Denaturierungsschritt bei 94°C für 30 Sekunden, ein Schritt zum Binden der Primer an die DNA bei 57°C für 30 Sekunden und der Extensions-Schritt zur Verlängerung der Primer bei 72°C für 60 sec.

Nach dem abschliessenden Extensions-Schritt für 5 min bei 72°C wurde der PCR-Ansatz einer Agarosegel-Elektrophorese (0,8% Agarose) unterworfen. Die Identifizierung eines DNA-Fragmentes von 1080 bp Länge zeigt hierbei die Integration des Promotors Pg3 vor lpdA an, wohingegen ein DNA-Fragment von 591 bp Länge den wildtypischen Status des Ausgangsstammes anzeigt.

Auf diese Weise wurden Mutanten identifiziert, welche die Mutation Pg3_lpdA in Form einer Integration vorliegen haben, wobei einer der erhaltenen Stämme C. glutamicum DM1547_Pg3_lpdA genannt wurde.

### Beispiel 5:

### Herstellung der Stämme DM1547 / pZ8-1, DM1547 / pZ8-1::lpdA, DM1933 / pZ8-1 und DM1933 / pZ8-1::lpdA

Die Plasmide pZ8-1 und pZ8-1::lpdA (Beispiel 3) wurden mit der Elektroporationsmethode von Liebl et al. (FEMS Microbiological Letters, 53:299-303 (1989)) in Corynebacterium glutamicum DM1547 und DM1933 elektroporiert. Die Selektion von Plasmid-tragenden Zellen erfolgte durch Ausplattieren des Elektroporationsansatzes auf LB Agar (Sambrook et al., Molecular cloning: a laboratory manual. 2nd Ed. Cold Spring Harbor Laboratory Press, Cold Spring Harbor, N.Y., 1989), der mit 25 mg/l Kanamycin supplementiert worden war. Plasmid DNA wurde nach den üblichen Methoden aus jeweils einer Transformante isoliert (Peters-Wendisch et al., 1998, Microbiology, 144, 915-927) und durch Restriktionsspaltung mit anschließender Agarosegel-Elektrophorese überprüft.

Die erhaltenen Stämme wurden DM1547 / pZ8-1 und DM1547 /pZ8-1::lpdA sowie DM1933 / pZ8-1 und DM1933 / pZ8-1::lpdA genannt.

### Beispiel 6:

### Herstellung von L-Lysin mit den Stämmen DM1547_Pg3_lpdA und DM1547

Der in Beispiel 4 erhaltenen *C*. *glutamicum* Stamm DM1547_Pg3_lpdA und der Ausgangsstamm DM1547 wurden in einem zur Produktion von Lysin geeigneten Nährmedium kultiviert und der Lysingehalt im Kulturüberstand bestimmt.

Dazu wurden die Klone zunächst auf Hirn-Herz-Agarplatten (Merck, Darmstadt, Deutschland) für 24 Stunden bei 33°C vermehrt. Ausgehend von diesen Agarplattenkulturen wurde je eine Vorkultur angeimpft (10 ml Medium im 100 ml Erlenmeyerkolben). Als Medium für die Vorkultur wurde das Medium MM verwendet. Die Vorkultur wurde 24 Stunden bei 33°C und 240 rpm auf einem Schüttler inkubiert. Von dieser Vorkultur wurde eine Hauptkultur angeimpft, so dass die Anfangs-OD (660 nm) der Hauptkultur 0,1 OD betrug. Für die Hauptkultur wurde ebenfalls das Medium MM verwendet.

Tabelle 1 gibt eine Übersicht über die Zusammensetzung des verwendeten Kultivierungsmediums.

**Tabelle 1: Medium MM**

| | |
|---|---|
| CSL (Corn Steep Liquor) | 5 g/l |
| MOPS (Morpholinopropansufonsäure) | 20 g/l |
| Glucose (getrennt autoklaviert) | 50 g/l |

| Salze: | |
|---|---|
| (NH₄)₂SO₄) | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0 mg/l |
| Biotin (sterilfiltriert) | 0,3 mg/l |
| Thiamin * HCl (sterilfiltriert) | 0,2 mg/l |
| CaCO₃ | 25 g/l |

CSL (Corn Steep Liquor), MOPS (Morpholinopropansulfonsäure) und die Salzlösung wurden mit Ammoniakwasser auf pH 7 eingestellt und autoklaviert. Anschließend wurden die sterilen Substrat- und Vitaminlösungen sowie das trocken autoklavierte CaCO₃ zugesetzt.

Die Kultivierung erfolgte in Volumina von 10 ml, die in 100 ml Erlenmeyerkolben mit Schikanen enthalten waren. Die Temperatur betrug bei 33°C, die Umdrehungszahl war 250 rpm und die Luftfeuchte 80%.

Nach 40 Stunden wurde die optische Dichte (OD) bei einer Meßwellenlänge von 660 nm mit dem Biomek 1000 (Beckmann Instruments GmbH, München) bestimmt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

In Tabelle 2 ist das Ergebnis des Versuchs dargestellt.

**Tabelle 2: Herstellung von L-Lysin**

| Stamm | L-Lysin HCl (g/l) | OD (660nm) |
|---|---|---|
| DM1547 | 19,0 | 12,3 |
| DM1547_Pg3_lpdA | 20,8 | 12,2 |

Alle Werte sind Mittelwerte von 3 unabhängigen Experimenten mit den genannten Stämmen

Das Ergebnis zeigt, dass die Ausbeute des gewünschten Produktes (L-Lysin) deutlich erhöht ist.

### Beispiel 7:

Herstellung von L-Lysin mit den Stämmen DM1547 /pZ8-1::lpdA, DM1547 / pZ8-1, DM1933 / pZ8-1 und DM1933 / pZ8-1::lpdA

Die in Beispiel 5 erhaltenen *C*. *glutamicum* Stämme DM1547 /pZ8-1::lpdA und DM1933 / pZ8-1::lpdA und die Kontrollstämme DM1547 / pZ8-1 und DM1933 / pZ8-1 wurden in einem zur Produktion von Lysin geeigneten Nährmedium kultiviert und der Lysingehalt im Kulturüberstand bestimmt.

Dazu wurden die Stämme zunächst auf Agarplatte mit dem entsprechenden Antibiotikum (Hirn-Herz Agar mit Kanamycin (25 mg/l)) für 24 Stunden bei 33°C inkubiert. Ausgehend von diesen Agarplattenkulturen wurde je eine Vorkultur angeimpft (10 ml Medium im 100 ml Erlenmeyerkolben). Als Medium für die Vorkultur wurde das Medium MM (Tabelle 1 aus Beispiel 6) verwendet welches mit Kanamycin (25 mg/l) versetzt wurde. Die Vorkultur wurde 24 Stunden bei 33°C und 240 rpm auf einem Schüttler inkubiert. Von dieser Vorkultur wurde eine Hauptkultur angeimpft, so dass die Anfangs-OD (660 nm) der Hauptkultur 0,1 OD betrug. Für die Hauptkultur wurde ebenfalls das Medium MM verwendet.

Die Kultivierung erfolgte in Volumina von 10 ml, die in 100 ml Erlenmeyerkolben mit Schikanen enthalten waren. Die Temperatur betrug bei 33°C, die Umdrehungszahl war 250 rpm und die Luftfeuchte 80%.

Nach 40 Stunden wurde die optische Dichte (OD) bei einer Meßwellenlänge von 660 nm mit dem Biomek 1000 (Beckmann Instruments GmbH, München) bestimmt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

In Tabelle 3 ist das Ergebnis des Versuchs dargestellt.

**Tabelle 3: Herstellung von L-Lysin**

| Stamm | L-Lysin HCl (g/l) | OD (660nm) |
|---|---|---|
| DM1933 / pZ8-1 | 12,2 | 13,1 |
| DM1933 / pZ8-1::lpdA | 14,5 | 13,0 |
| DM1547 / pZ8-1 | 18,5 | 12,5 |
| DM1547 / p28-1::lpdA | 21,0 | 12,3 |

Alle Werte sind Mittelwerte von 3 unabhängigen Experimenten mit den genannten Stämmen

Das Ergebnis zeigt, dass die Ausbeute des gewünschten Produktes (L-Lysin) deutlich erhöht ist.

### Beispiel 8:

### Transformation des Stammes ECM3 mit den Plasmiden pZ8-1 und pZ8-1_lpdA

Der L-Methionin produzierende *E*. *coli* Stamm ECM3 basiert auf dem wildtypischen K12-Stamm MG1655. Der Stamm ECM3 trägt wie in EP2205754A2, EP2326724A1 und EP12156052.8 beschrieben ein feedbackresistentes metA-Allel, eine Deletion der Gene metJ, yncA, pykA, pykF und purU, eine Variante des spoT-Gens, sowie jeweils eine Promotorverstärkung vor den Genen metH, metF, gcvT, cysP und cysJ.

Der Stamm ECM3 wurde mit den Plasmiden pZ8-1 und pZ8-1_lpda aus Beispiel 3 transformiert und die Transformanden mit 20 mg/l Kanamycin selektioniert. Die resultierenden Stämme wurden mit ECM3/pZ8-1 und ECM3/pZ8-1_lpdA bezeichnet.

### Beispiel 9:

### Herstellung von L-Methionin mit den Stämmen ECM3 und ECM3/pZ8-1_lpdA

Die Leistungsfähigkeit der *E.coli* L-Methionin-Produktionsstämme ECM3 und ECM3/pZ8-1_lpda wurde durch Produktionsversuche in 100 ml Erlenmeyer-Kolben bewertet. Als Vorkulturen wurden jeweils 10 ml Vorkulturmedium (10% LB-Medium mit 2,5 g/l Glucose und 90% Minimalmedium PC1 (Tabelle 4)) mit 100µl Zellkultur beimpft und 10 Stunden bei 37°C kultiviert. Hiermit wurden anschließend je 10 ml PC1-Minimalmedium auf eine OD 600 nm von 0,2 (Eppendorf Bio-Photometer; Eppendorf AG, Hamburg, Deutschland) beimpft und für 24 Stunden bei 37°C kultiviert. Die extrazelluläre L-Methionin-Konzentration wurde mit einem Aminosäureanalysator (Sykam GmbH, Eresing, Deutschland) durch Ionenaustauschchromatographie und Nachsäulenderivatisierung mit Ninhydrindetektion bestimmt. Die extrazelluläre Glucose-Konzentration wurde mit einem YSI 2700 Select Glucose Analyzer (YSI Life Sciences, Yellow Springs, Ohio, USA) bestimmt. Die Ergebnisse sind in Tabelle 5 dargestellt. Nach 48 Stunden war die Glucose in beiden Kulturen vollständig verbraucht.

**Tabelle 4: Minimal Medium PC1**

| Substanz | Konzentration |
|---|---|
| ZnSO4 * 7 H2O | 4 mg/l |
| CuCl2 * 2 H2O | 2 mg/l |
| MnSO4 * H2O | 20 mg/l |
| H3BO3 | 1 mg/l |
| Na2MoO4 * 2 H2O | 0,4 mg/l |
| MgSO4 * 7 H2O | 1 g/l |
| Citronensäure * 1 H2O | 6,56 g/l |
| CaCl2 * 2 H2O | 40 mg/l |
| K2HPO4 | 8,02 g/l |
| Na2HPO4 | 2 g/l |
| (NH4)2HPO4 | 8 g/l |
| NH4Cl | 0,13 g/l |
| (NH4)2SO | 5, g/l |
| MOPS | 5 g/l |
| NaOH 10M | auf pH 6,8 eingestellt |
| | |
| FeSO4 * 7 H2O | 40 mg/l |
| Thiaminhydrochlorid | 10 mg/l |
| Vitamin B12 | 10 mg/l |
| Glucose | 10 g/l |
| Kanamycin | 50 mg/l |

**Tabelle 5: L-Methioninkonzentrationen in den Fermentationsbrühen der untersuchten E. coli Stämme**

| Stamm | OD(600nm) | L-Methionin (g/l) |
|---|---|---|
| ECM3/pZ8-1 | 3,38 | 0,40 |
| ECM3/pZ8-1_lpdA | 3, 17 | 0,57 |

Alle Werte sind Mittelwerte von 3 unabhängigen Experimenten mit den genannten Stämmen

Das Ergebnis zeigt, dass die Ausbeute des gewünschten Produktes (L-Methionin) deutlich erhöht ist.

### Figur 1: Karte des Plasmids pK18msb_Pg3_lpdA

Die verwendeten Abkürzungen und Bezeichnungen haben folgende Bedeutung. Bei der Angabe der Basenpaarzahlen handelt es sich um Näherungswerte, die im Rahmen der Reproduzierbarkeit von Messungen erhalten werden.

| KanR: | Kanamycin Resistenz-Gen |
|---|---|
| XmaI | Schnittstelle des Restriktionsenzyms XmaI |
| EcoRI | Schnittstelle des Restriktionsenzyms EcoRI |
| XbaI | Schnittstelle des Restriktionsenzyms XbaI |
| sacB: | sacB-Gen |
| oriV: | Replikationsursprung V |

### Figur 2: Karte des Plasmids p28-1::lpdA

Die verwendeten Abkürzungen und Bezeichnungen haben folgende Bedeutung. Bei der Angabe der Basenpaarzahlen handelt es sich um Näherungswerte, die im Rahmen der Reproduzierbarkeit von Messungen erhalten werden.

| | |
|---|---|
| Km | Kanamycin Resistenz-Gen |
| EcoRI | Schnittstelle des Restriktionsenzyms EcoRI |
| SalII | Schnittstelle des Restriktionsenzyms SalI |
| Ptac | tac-Promoter |
| rep | Replikationsursprung Escherichia coli |
| TrrnB | rrnB Terminator |

## Patentansprüche

1. Verfahren zur fermentativen Herstellung einer organisch-chemischen Verbindung unter Verwendung eines Mikroorganismus, **dadurch gekennzeichnet, dass** man folgende Schritte durchführt:
a) Fermentation eines eine organisch-chemische Verbindung produzierenden Mikroorganismus, wobei in dem Mikroorganismus ein Polynukleotid, das für ein Polypeptid kodiert dessen Aminosäuresequenz wenigstens 80% identisch ist mit der Aminosäuresequenz von SEQ ID NO:2, überexprimiert vorliegt und wobei die Fermentation in einem Fermentationsmedium unter Bildung einer Fermentationsbrühe abläuft,
b) Anreichern der organisch-chemischen Verbindung in der Fermentationsbrühe aus a).

2. Verfahren zur Herstellung einer organisch-chemischen Verbindung nach Anspruch 1, **dadurch gekennzeichnet, dass** in dem Mikroorganismus ein Polynukleotid, das für ein Polypeptid kodiert dessen Aminosäuresequenz wenigstens 95% identisch ist mit der Aminosäuresequenz von SEQ ID NO:2, überexprimiert vorliegt

3. Verfahren zur Herstellung einer organisch-chemischen Verbindung nach Anspruch 1 oder 2, **dadurch gekennzeichnet, dass** das kodierte Polypeptid die Aktivität einer Transhydrogenase aufweist.

4. Verfahren zur Herstellung einer organisch-chemischen Verbindung nach einem der Ansprüche 1 bis 3, **dadurch gekennzeichnet, dass** das kodierte Polypeptid die Aminosäuresequenz von SEQ ID NO:2 umfasst.

5. Verfahren zur Herstellung einer organisch-chemischen Verbindung nach Anspruch 1, **dadurch gekennzeichnet, dass** die Herstellung der organisch chemischen Verbindung gegenüber der Fermentation eines Mikroorganismus, in dem das Polynukleotid, das für ein Polypeptid kodiert dessen Aminosäuresequenz wenigstens 80% identisch ist mit der Aminosäuresequenz von SEQ ID NO:2, nicht überexprimiert vorliegt, um mindestens 0,5% erhöht ist.

6. Das Verfahren zur Herstellung einer organisch-chemischen Verbindung nach einem der Ansprüche 1 bis 5, **dadurch gekennzeichnet, dass** die Überexpression durch eine oder mehrere der Maßnahmen erzielt wird ausgewählt aus der Gruppe
a) die Expression des Gens steht unter der Kontrolle eines Promotors, der in dem für das Verfahren verwendeten Mikroorganismus stärker ist als der native Promotor des Gens;
b) Erhöhung der Kopienzahl des Gens kodierend für ein Polypeptid mit der Aktivität einer Transhydrogenase; vorzugsweise durch Einfügen des Gens in Plasmiden mit erhöhter Kopienzahl und/oder durch Integration des Gens in das Chromosom des Mikroorganismus in mindestens einer Kopie;
c) die Expression des Gens erfolgt unter Verwendung einer Ribosomen-Bindestelle, die in dem für das Verfahren verwendeten Mikroorganismus stärker ist als die ursprüngliche Ribosomen-Bindestelle des Gens;
d) die Expression des Gens erfolgt unter Optimierung der Kodon-Verwendung (codon usage) des für das Verfahren verwendeten Mikroorganismus;
e) die Expression des Gens erfolgt unter Reduzierung von mRNA Sekundärstrukturen in der vom Gen transkribierten mRNA;
f) die Expression des Gens erfolgt unter Eliminierung von RNA-Polymerase-Terminatoren in der vom Gen transkribierten mRNA;
g) die Expression des Gens erfolgt unter Verwendung mRNA-stabilisierender Sequenzen in der vom Gen transkribierten mRNA.

7. Verfahren zur Herstellung einer organisch-chemischen Verbindung nach einem der Ansprüche 1 bis 6, **dadurch gekennzeichnet, dass** es sich bei der organisch-chemischen Verbindung um eine L-Aminosäure ausgewählt aus der Gruppe L-Threonin, L-Isoleucin, L-Lysin, L-Methionin, L-Ornithin, L-Prolin, L-Valin, L-Leucin und L-Tryptophan handelt.

8. Das Verfahren zur Herstellung einer organisch-chemischen Verbindung nach einem der Ansprüche 1 bis 7, **dadurch gekennzeichnet, dass** es sich um einen eine organisch-chemische Verbindung ausscheidenden Mikroorganismus der Gattung Corynebacterium oder Escherichia handelt.

9. Das Verfahren zur Herstellung einer organisch-chemischen Verbindung nach einem der Ansprüche 1 bis 8, **dadurch gekennzeichnet, dass** es sich um einen eine organisch-chemische Verbindung ausscheidenden Mikroorganismus der Art Corynebacterium glutamicum oder der Art Escherichia coli handelt.

10. Das Verfahren zur Herstellung einer organisch-chemischen Verbindung nach einem der Ansprüche 1 bis 6, **dadurch gekennzeichnet, dass** es sich um ein Verfahren ausgewählt aus der Gruppe Satzverfahren, Zulaufverfahren, repetitives Zulaufverfahren und kontinuierliches Verfahren handelt.

11. Das Verfahren nach Anspruch 7, **dadurch gekennzeichnet, dass** man aus der organisch-chemischen Verbindunghaltigen Fermentationsbrühe die organisch-chemische Verbindung oder ein flüssiges oder festes organisch-chemischen Verbindung-haltiges Produkt gewinnt.

12. Mikroorganismus, der eine organisch-chemische Verbindung produziert, worin ein Polynukleotid, das für ein Polypeptid kodiert, dessen Aminosäuresequenz ≥ 80% identisch ist mit der Aminosäuresequenz von SEQ ID NO:2, überexprimiert vorliegt.

13. Mikroorganismus nach Anspruch 12 **dadurch gekennzeichnet, dass** die Aminosäuresequenz des kodierten Polypeptids ≥ 95% identisch ist mit der Aminosäuresequenz von SEQ ID NO:2.

14. Mikroorganismus nach Anspruch 12 **dadurch gekennzeichnet, dass** das kodierte Polypeptid die Aminosäuresequenz von SEQ ID NO:2 umfasst.

15. Mikroorganismus nach einem der Ansprüche 12 bis 14, ausgewählt aus der Gruppe der Gattung Corynebacterium und der Bakterien der Familie Enterobacteriacae, wobei die Art Corynebacterium glutamicum bevorzugt wird.

16. Mikroorganismus nach einem der Ansprüche 12 bis 15, **dadurch gekennzeichnet, dass** das überexprimierte Polynukleotid die Aktivität einer Transhydrogenase hat.
